# EUROPEAN PATENT APPLICATION

(11) **EP 2 096 105 A1**
(43) Date of publication of application: **02.09.2009**
(21) Application number: 08382008.4
(22) Date of filing: 28.02.2008
(51) Int. Cl.: C07C 275/40, C07D 215/26, A61K 31/17, A61K 31/4704, A61P 9/00, A61P 11/00, A61P 27/06

(54) **Derivatives of 4-(2-amino-1-hydroxyethyl)phenol as agonists of the b2 adrenergic receptor**

(71) Applicant: Laboratorios Almirall, S.A., 08022 Barcelona (ES)
(72) Inventor: Puig Duran, Carlos, 08024, Barcelona (ES); Perez Crespo, Daniel, 08024, Barcelona (ES); Crespo Crespo, María Isabel, 08036, Barcelona (ES); Sole Feu, Laia, 08950, Esplugues de Llobregat, Barcelona (ES); Prat Quinones, Maria, 08003, Barcelona (ES)
(74) Representative: Elzaburu Marquez, Alberto

(57) **Abstract**

The present invention provides a compound of formula (I): 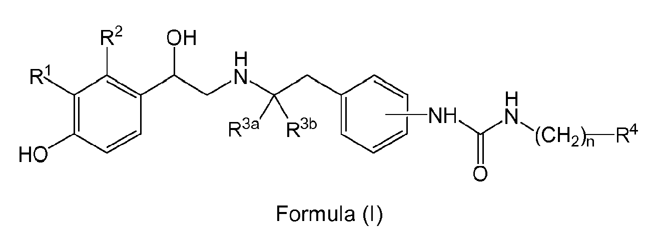 wherein:
R¹ is a group selected from -CH₂OH,-NHCOH and
R² is a hydrogen atom; or
R¹ together with R² form the group -NHC(O)CH=CH-, wherein the nitrogen atom is bound to the carbon atom in the phenyl ring holding R¹ and the carbon atom is bound to the carbon atom in the phenyl ring holding R²,
R^{3a} and R^{3b} are independently selected from the group consisting of hydrogen atoms and C₁₋₄ alkyl groups
n is an integer selected from 0 to 6,
R⁴ is selected from the group consisting of an optionally substituted monocyclic or polycyclic C₃₋₁₀ cycloalkyl group, an optionally substituted monocyclic C₅₋₁₀ aryl group and, a methyl group which is substituted with one or more substituents selected from C₅₋₁₀ aryl and C₅₋₁₀ aryloxy groups,
wherein the monocyclic or polycyclic C₃₋₁₀ cycloalkyl and the monocyclic C₅₋₁₀ aryl groups independently are optionally substituted with one or more substituents selected from halogen atoms, C₁₋₄ alkyl, C₁₋₄ alkoxy, C₅₋₁₀ aryl and C₅₋₁₀ aryloxy groups,

or a pharmaceutically-acceptable salt or solvate or stereoisomer thereof.

## Description

### FIELD OF THE INVENTION

The present invention is directed to novel β2 adrenergic receptor agonists. The invention is also directed to pharmaceutical compositions comprising such compounds, methods of using such compounds to treat diseases associated with β2 adrenergic receptor activity, and processes and intermediates useful for preparing such compounds.

### BACKGROUND OF THE INVENTION

β2 adrenergic receptor agonists are recognized as effective drugs for the treatment of pulmonary diseases such as asthma and chronic obstructive pulmonary disease (including chronic bronchitis and emphysema). β2 adrenergic receptor agonists are also useful for treating pre-term labor, glaucoma and are potentially useful for treating neurological disorders and cardiac disorders.

In spite of the success that has been achieved with certain β2 adrenergic receptor agonists, current agents possess less than desirable potency, selectivity, onset, and/or duration of action. Thus, there is a need for additional β2 adrenergic receptor agonists having improved properties. Preferred agents may possess, among other properties, improved potency, selectivity, onset, improved safety margins, improved therapeutic window and/or duration of action.

### SUMMARY OF THE INVENTION

The invention provides novel compounds that possess β2 adrenergic receptor agonist activity. Accordingly, there is provided a compound of the invention which is of formula (I): 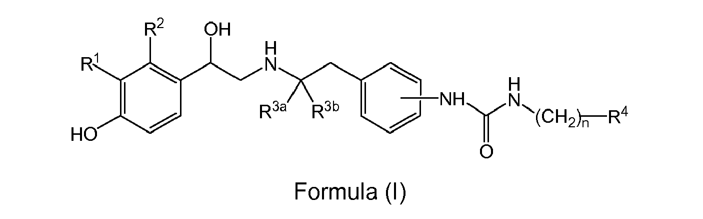 wherein:
R¹ is a group selected from -CH₂OH,-NHCOH and
R² is a hydrogen atom; or
R¹ together with R² form the group -NHC(O)CH=CH-, wherein the nitrogen atom is bound to the carbon atom in the phenyl ring holding R¹ and the carbon atom is bound to the carbon atom in the phenyl ring holding R²
R^{3a} and R^{3b} are independently selected from the group consisting of hydrogen atoms and C₁₋₄ alkyl groups
n is an integer selected from 0 to 6,
R⁴ is selected from the group consisting of an optionally substituted monocyclic or polycyclic C₃₋₁₀ cycloalkyl group, an optionally substituted monocyclic C₅₋₁₀ aryl group and a methyl group which is substituted with one or more substituents selected from C₅₋₁₀ aryl and C₅₋₁₀ aryloxy groups,
wherein the monocyclic or polycyclic C₃₋₁₀ cycloalkyl and the monocyclic C₅₋₁₀ aryl groups independently are optionally substituted with one or more substituents selected from halogen atoms, C₁₋₄ alkyl, C₁₋₄ alkoxy, C₅₋₁₀ aryl and C₅₋₁₀ aryloxy groups, or a pharmaceutically-acceptable salt or solvate or stereoisomer thereof.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

The invention also provides a method of treating a disease or condition associated with β2 adrenergic receptor activity (e.g. a pulmonary disease, such as asthma or chronic obstructive pulmonary disease, pre-term labor, glaucoma, a neurological disorder, a cardiac disorder, or inflammation) in a mammal, comprising administering to the mammal, a therapeutically effective amount of a compound of the invention. The invention further provides a method of treatment comprising administering a therapeutically effective amount of a combination of a compound of the invention together with one or more other therapeutic agents.

In separate and distinct aspects, the invention also provides synthetic processes and intermediates described herein, which are useful for preparing compounds of the invention.

The invention also provides a compound of the invention as described herein for use in medical therapy, as well as the use of a compound of the invention in the manufacture of a formulation or medicament for treating a disease or condition associated with β2 adrenergic receptor activity (e.g. a pulmonary disease, such as asthma or chronic obstructive pulmonary disease, pre-term labor, glaucoma, a neurological disorder, a cardiac disorder, or inflammation) in a mammal.

### DETAILED DESCRIPTION OF THE INVENTION

When describing the compounds, compositions and methods of the invention, the following terms have the following meanings, unless otherwise indicated.

As used herein the term C₁₋₄ alkyl embraces optionally substituted, linear or branched radicals having 1 to 4 carbon atoms. Examples include methyl, ethyl, n-propyl, i-propyl, n-butyl, sec-butyl, t-butyl,

A said optionally substituted alkyl group is typically unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, substituents on an alkyl group are themselves unsubstituted. Preferred optionally substituted alkyl groups are unsubstituted or substituted with 1, 2 or 3 fluorine atoms.

As used herein, the term C₁₋₄ alkoxy (or alkyloxy) embraces optionally substituted, linear or branched oxy-containing radicals each having alkyl portions of 1 to 4 carbon atoms.

An alkoxy group is typically unsubstituted or substituted with 1, 2 or 3 substituents which may be the same or different. The substituents are preferably selected from halogen atoms, preferably fluorine atoms, hydroxy groups and C₁₋₄ alkoxy groups. Typically, the substituents on an alkoxy group are themselves unsubstituted.

Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, the term C₅-C₁₀ aryl radical embraces typically a C₅-C₁₄ monocyclic or polycyclic aryl radical such as phenyl, naphthyl, anthranyl and phenanthryl. Phenyl is preferred.

As used herein, the term C₃₋₁₀ cycloalkyl group embraces saturated carbocyclic radicals monocyclic or polycyclic ring having from 3 to 10 carbon atoms. Examples include cyclopropyl, cyclobutyl, cyclopentyl, cyclohexyl and adamantyl. It is preferably cyclopropyl, cyclopentyl and cyclohexyl.

As used herein, an aryloxy group is typically a said aryl group attached to an oxygen atom.

As used herein, the term halogen atom embraces chlorine, fluorine, bromine or iodine atoms typically a fluorine, chlorine or bromine atom. The term halo when used as a prefix has the same meaning.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with β2 adrenergic receptor activity" includes all disease states and/or conditions that are acknowledged now, or that are found in the future, to be associated with β2 adrenergic receptor activity. Such disease states include, but are not limited to, pulmonary diseases, such as asthma and chronic obstructive pulmonary disease (including chronic bronchitis and emphysema), as well as neurological disorders and cardiac disorders. β2 adrenergic receptor activity is also known to be associated with pre-term labor (see International Patent Application Publication Number WO 98/09632), glaucoma and some types of inflammation (see International Patent Application Publication Number WO 99/30703 and Patent Application Publication Number EP 1 078 629).

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, fumaric, gluconic, glutamic, hydrobromic, hydrochloric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

The term "solvate" refers to a complex or aggregate formed by one or more molecules of a solute, i.e. a compound of the invention or a pharmaceutically-acceptable salt thereof, and one or more molecules of a solvent. Such solvates are typically crystalline solids having a substantially fixed molar ratio of solute and solvent. Representative solvents include by way of example, water, methanol, ethanol, isopropanol, acetic acid, and the like. When the solvent is water, the solvate formed is a hydrate.

It will be appreciated that the term "or a pharmaceutically-acceptable salt or solvate of stereoisomer thereof" is intended to include all permutations of salts, solvates and stereoisomers, such as a solvate of a pharmaceutically-acceptable salt of a stereoisomer of a compound of formula (I).

The term "amino-protecting group" refers to a protecting group suitable for preventing undesired reactions at an amino nitrogen. Representative amino-protecting groups include, but are not limited to, formyl; acyl groups, for example alkanoyl groups such as acetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl; silyl groups such as trimethylsilyl (TMS) and tert-butyldimethylsilyl (TBS); and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The compounds of the invention contain at least a chiral center. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

In an embodiment of the present invention, R¹ together with R² form the group - NH-C(O)-CH=CH-, wherein the nitrogen atom is bound to the carbon atom in the phenyl ring holding R¹ and the carbon atom is bound to the carbon atom in the phenyl ring holding R²

In another embodiment of the present invention, R^{3a} and R^{3b} are independently selected from the group consisting of hydrogen atoms and methyl groups. More preferably R^{3a} represents a hydrogen atom and R^{3b} is selected from the group consisting of hydrogen atoms and methyl groups

In a still another embodiment of the compounds of formula (I), n has a value of 0 to 3, more preferably n has a value of 0 or 1.

In an another embodiment of the present invention, R⁴ is selected from the group consisting of monocyclic or polycyclic C₄₋₁₀ cycloalkyl, a phenyl group and a methyl group which is substituted with one or two substituents selected from a phenyl and phenyloxy groups and wherein the cycloalkyl and the phenyl groups independently are optionally substituted with one or more substituents selected from fluorine and chlorine atoms, methoxy, phenyl and phenoxy groups. More preferably, R⁴ is selected from the group consisting of cyclohexyl, 1-adamantyl, phenyl and CH(Ph)₂- groups, wherein the phenyl group is optionally substituted with one or more substituents selected from methoxy, phenyl and phenoxy groups and most preferably R⁴ is selected from the group consisting of a phenyl and CH(Ph)₂- groups, wherein the phenyl group is optionally substituted with one or two substituents selected from methoxy, and phenyl groups.

In another embodiment, the present invention provides compounds of formula (IA):

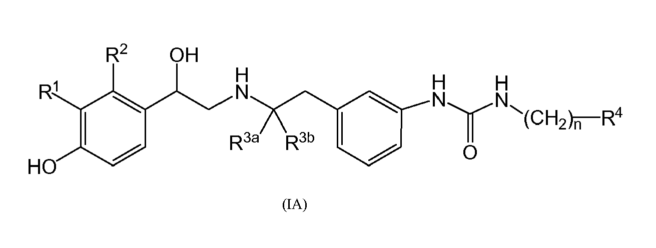

In a still prefered embodiment, the present invention provides compounds of formula (IA) wherein R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R², R^{3a} represents a hydrogen atom and R^{3b} is selected from the group consisting of hydrogen atom and methyl group, n has a value of 0 or 1 and R⁴ is selected from the group consisting of a phenyl group and a CH(Ph)₂- group, wherein the phenyl group is optionally substituted with one or two substituents selected from methoxy, and phenyl groups.

Particular individual compounds of the invention include:
1. N-benzyl-N'-{3-[(2*R*,*S*)-2-({(2*R*,*S*)-2-hydroxy-2-[4-hydroxy-3-(hydroxymethyl)-phenyl]ethyl}aminO)propyl]phenyl}urea
2. N-benzyl-N'-{4-[(2*R*,*S*)-2-({(2*R*,*S*)-2-hydroxy-2-[4-hydroxy-3-(hydroxymethyl)-phenyl]ethyl}amino)propyl]phenyl}urea
3. N-benzyl-N'-[3-((2*R*,*S*)-2-{[(2*R*,*S*)-2-hydroxy-2-(8-hydroxy-2-oxo-1,2-dihydroquinolin-5-yl)ethyl]amino}propyl)phenyl]urea
4. N-{3-[(2*R*,*S*)-2-({(2*R*,*S*)-2-hydroxy-2-[4-hydroxy-3-(hydroxymethyl)phenyl]-ethyl}amino)propyl]phenyl}-N'-(2-methoxybenzyl)urea
5. N-(2,6-dimethoxybenzyl)-N'-{3-[(2*R*,*S*)-2-({(2*R*)-2-hydroxy-2-[4-hydroxy-3-(hydroxymethyl)phenyl]ethyl}amino)propyl]phenyl}urea
6. N-[3-((2*R*,*S*)-2-{[(2*R*,*S*)-2-hydroxy-2-(8-hydroxy-2-oxo-1,2-dihydroquinolin-5-yl)ethyl]amino}propyl)phenyl]-N'-(2-methoxybenzyl)urea
7. N-benzyl-N'-{3-[2-({(2*R*,*S*)-2-hydroxy-2-[4-hydroxy-3-(hydroxymethyl)phenyl]-ethyl}amino)ethyl]phenyl}urea
8. N-benzyl-N'-[3-(2-{[(2*R*,*S*)-2-hydroxy-2-(8-hydroxy-2-oxo-1,2-dihydroquinolin-5-yl)ethyl]amino}ethyl)phenyl]urea
9. N-[3-(2-{[(2*R*,*S*)-2-hydroxy-2-(8-hydroxy-2-oxo-1,2-dihydroquinolin-5-yl)ethyl]-amino}ethyl)phenyl]-N'-(2-methoxybenzyl)urea
10. N-(4-fluorobenzyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
11. N-[3-(2-{[(2*R*,*S*)-2-hydroxy-2-(8-hydroxy-2-oxo-1,2-dihydroquinolin-5-yl)ethyl]-amino}ethyl)phenyl]-N'-(2-phenylethyl)urea
12. N-[3-(2-{[(2*R*,*S*)-2-hydroxy-2-(8-hydroxy-2-oxo-1,2-dihydroquinolin-5-yl)ethyl]-amino}ethyl)phenyl]-N'-(3-phenylpropyl)urea
13. N-{3-[2-({(2*R*,*S*)-2-hydroxy-2-[4-hydroxy-3-(hydroxymethyl)phenyl]ethyl}-amino)ethyl]phenyl}-N'-(3-phenylpropyl)urea
14. N-(diphenylmethyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
15. N-[3-(2-{[(2*R*,*S*)-2-hydroxy-2-(8-hydroxy-2-oxo-1,2-dihydroquinolin-5-yl)ethyl]-amino}ethyl)phenyl]-N'-phenylurea
16. N-1-adamantyl-N'-[3-(2-{[(2*R*,*S*)-2-hydroxy-2-(8-hydroxy-2-oxo-1,2-dihydroquinolin-5-yl)ethyl]amino}ethyl)phenyl]urea
17. N-(2,6-dichlorobenzyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
18. N-biphenyl-2-yl-N'-[3-(2-{[(2*R*,*S*)-2-hydroxy-2-(8-hydroxy-2-oxo-1,2-dihydroquinolin-5-yl)ethyl]amino}ethyl)phenyl]urea
19. N-cyclohexyl-N'-[3-(2-{[(2*R*,*S*)-2-hydroxy-2-(8-hydroxy-2-oxo-1,2-dihydroquinolin-5-yl)ethyl]amino}ethyl)phenyl]urea
20. N-[3-(2-{[(2*R*,*S*)-2-hydroxy-2-(8-hydroxy-2-oxo-1,2-dihydroquinolin-5-yl)ethyl]-amino}ethyl)phenyl]-N'-(4-phenylbutyl)urea
21. N-[3-(2-{[(2*R*,*S*)-2-hydroxy-2-(8-hydroxy-2-oxo-1,2-dihydroquinolin-5-yl)ethyl]-amino}ethyl)phenyl]-N'-(4-methoxyphenyl)urea
22. N-biphenyl-4-yl-N'-[3-(2-{[(2*R*,*S*)-2-hydroxy-2-(8-hydroxy-2-oxo-1,2-dihydroquinolin-5-yl)ethyl]amino}ethyl)phenyl]urea
23. N-(1-adamantylmethyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
24. N-[3-(2-{[(2*R*,*S*)-2-hydroxy-2-(8-hydroxy-2-oxo-1,2-dihydroquinolin-5-yl)ethyl]-amino}ethyl)phenyl]-N'-(3-phenoxyphenyl)urea
25. N-[3-(2-{[(2*R*)-2-hydroxy-2-(8-hydroxy-2-oxo-1,2-dihydroquinolin-5-yl)ethyl]-amino}ethyl)phenyl]-N'-(3-phenylpropyl)urea
26. N-(diphenylmethyl)-N'-[3-(2-{[(2*R*)-2-hydroxy-2-(8-hydroxy-2-oxo-1,2-dihydroquinolin-5-yl)ethyl]amino}ethyl)phenyl]urea
27. *N*-biphenyl-2-yl-N'-[3-(2-{[(2*R*)-2-hydroxy-2-(8-hydroxy-2-oxo-1,2-dihydroquinolin-5-yl)ethyl]amino}ethyl)phenyl]urea
28. *N*-biphenyl-2-yl-N'-{3-[2-({(2*R*)-2-[3-(formylamino)-4-hydroxyphenyl]-2-hydroxyethyl}amino)ethyl]phenyl}urea
29. *N*-biphenyl-2-yl-*N*'-[3-(2-{[(2*R*)-2-hydroxy-2-(8-hydroxy-2-oxo-1,2-dihydroquinolin-5-yl)ethyl]amino}propyl)phenyl]urea, (isomer A)
30. *N*-biphenyl-2-yl-*N*'-{3-[2-({(2*R*)-2-hydroxy-2-[4-hydroxy-3-(hydroxymethyl)phenyl]-ethyl}amino)propyl]phenyl}urea, (isomer B)
31. *N*-biphenyl-2-yl-*N*'-{3-[2-({(2*R*)-2-hydroxy-2-[4-hydroxy-3-(hydroxymethyl)phenyl]-ethyl}amino)propyl]phenyl}urea, (isomer A)
32. *N*-biphenyl-2-yl-*N*'-[3-(2-{[(2*R*)-2-hydroxy-2-(8-hydroxy-2-oxo-1,2-dihydroquinolin-5-yl)ethyl]amino}propyl)phenyl]urea, (isomer B)
33. *N*-(4-tert-butylbenzyl)-*N*'-[3-(2-{[(2*R*,*S*)-2-hydroxy-2-(8-hydroxy-2-oxo-1,2-dihydro-quinolin-5-yl)ethyl]amino}ethyl)phenyl]urea
34. *N*-biphenyl-2-yl-*N*'-[4-(2-{[(2*R*)-2-hydroxy-2-(8-hydroxy-2-oxo-1,2-dihydroquinolin-5-yl)ethyl]amino}ethyl)phenyl]urea
35. *N*-[4-(2-{[(2*R*)-2-hydroxy-2-(8-hydroxy-2-oxo-1,2-dihydroquinolin-5-yl)ethyl]amino}-ethyl)phenyl]-*N*'-(3-phenylpropyl)urea
36. *N*-(diphenylmethyl)-*N*'-[4-(2-{[(2*R*)-2-hydroxy-2-(8-hydroxy-2-oxo-1,2-dihydro-quinolin-5-yl)ethyl]amino}ethyl)phenyl]urea

and pharmaceutically-acceptable salts and solvates thereof. Of particular interest are the compounds:
N-benzyl-N'-{4-[(2*R*,*S*)-2-({(2*R*,*S*)-2-hydroxy-2-[4-hydroxy-3-(hydroxymethyl)-phenyl]ethyl}amino)propyl]phenyl}urea
   N-[3-((2*R*,*S*)-2-{[(2*R*,*S*)-2-hydroxy-2-(8-hydroxy-2-oxo-1,2-dihydroquinolin-5-yl)ethyl]amino}propyl)phenyl]-N'-(2-methoxybenzyl)urea
   N-benzyl-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-[3-(2-{[(2*R*,*S*)-2-hydroxy-2-(8-hydroxy-2-oxo-1,2-dihydroquinolin-5-yl)ethyl]-amino}ethyl)phenyl]-N'-(2-methoxybenzyl)urea
   N-[3-(2-{[(2*R*,*S*)-2-hydroxy-2-(8-hydroxy-2-oxo-1,2-dihydroquinolin-5-yl)ethyl]-amino}ethyl)phenyl]-N'-(2-phenylethyl)urea
   N-[3-(2-{[(2*R*,*S*)-2-hydroxy-2-(8-hydroxy-2-oxo-1,2-dihydroquinolin-5-yl)ethyl]-amino}ethyl)phenyl]-N'-(3-phenylpropyl)urea
   N-(diphenylmethyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-1-adamantyl-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-(2,6-dichlorobenzyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-biphenyl-2-yl-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-cyclohexyl-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-(1-adamantylmethyl)-N'-[3-(2-{[(2*R*,*S*)-2-hydroxy-2-(8-hydroxy-2-oxo-1,2-dihydroquinolin-5-yl)ethyl]amino}ethyl)phenyl]urea
   N-[3-(2-{[(2*R*)-2-hydroxy-2-(8-hydroxy-2-oxo-1,2-dihydroquinolin-5-yl)ethyl]-amino}ethyl)phenyl]-N'-(3-phenylpropyl)urea
   N-(diphenylmethyl)-N'-[3-(2-{[(2*R*)-2-hydroxy-2-(8-hydroxy-2-oxo-1,2-dihydroquinolin-5-yl)ethyl]amino}ethyl)phenyl]urea
   *N*-biphenyl-2-yl-N'-[3-(2-{[(2*R*)-2-hydroxy-2-(8-hydroxy-2-oxo-1,2-dihydroquinolin-5-yl)ethyl]amino}ethyl)phenyl]urea
   *N*-biphenyl-2-yl-*N*'-[3-(2-{[(2*R*)-2-hydroxy-2-(8-hydroxy-2-oxo-1,2-dihydroquinolin-5-yl)ethyl]amino}propyl)phenyl]urea, (isomer B)
   *N*-[4-(2-{[(2*R*)-2-hydroxy-2-(8-hydroxy-2-oxo-1,2-dihydroquinolin-5-yl)ethyl]amino}-ethyl)phenyl]-*N*'-(3-phenylpropyl)urea

The invention also comprises pharmaceutical compositions comprising a therapeutically effective amount of a compound as hereinabove defined and a pharmaceutically acceptable carrier.

In an embodiment of the present invention the pharmaceutical composition further comprises a therapeutically effective amount of one or more other therapeutic agents.

It is also an embodiment of the present invention that the pharmaceutical composition is formulated for administration by inhalation.

The compounds of the present invention as hereinabove defined may also be combined with one or more other therapeutic agents, in particular one or more drugs selected from the group consisting of corticosteroids, antichlolinergic agents and PDE4 inhibitors.

The invention is also directed to a method of treating a disease or condition in a mammal associated with β2 adrenergic receptor activity, the method comprising administering to the mammal, a therapeutically effective amount of a pharmaceutical composition comprising a β2 adrenergic receptor agonist according to the present invention. It is of particular relevance the method applied to the treatment of a disease or condition which is a pulmonary disease, preferably asthma or chronic obstructive pulmonary disease.

The method of treating a disease can also be applied within the scope of the present invention to the treatment of a disease or condition selected from the group consisting of pre-term labor, glaucoma, neurological disorders, cardiac disorders, and inflammation.

The invention is also directed to the use of compunds of formula (I) in the manufacture of a medicament for the treatment of a pulmonary disease, preferably asthma or chronic obstructive pulmonary disease, pre-term labor, glaucoma, neurological disorders, cardiac disorders, and inflammation

The invention is also directed to compounds of formula (I) for use in the treatment of a pulmonary disease or condition, preferably asthma or chronic obstructive pulmonary disease, pre-term labor, glaucoma, neurological disorders, cardiac disorders, and inflammation

General Synthetic Procedures

The compounds of the invention can be prepared using the methods and procedures described herein, or using similar methods and procedures. It will be appreciated that
where typical or preferred process conditions (i.e., reaction temperatures, times, mole ratios of reactants, solvents, pressures, etc.) are given. Other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

One of the most convenient route for the preparation of compounds of formula (I) is depicted in Figure 1.

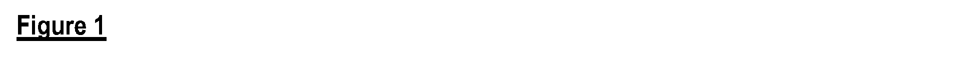

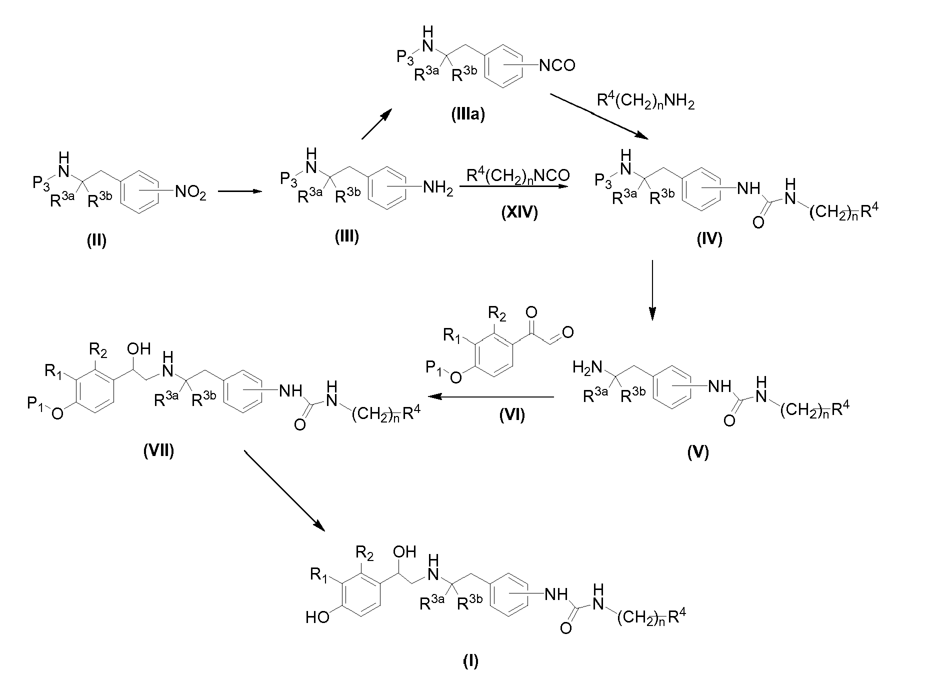

The known N-protected nitrophenethylamine of general formula (**II**) is reduced to the corresponding amino derivative of general formula (**III**). This transformation is best carried out by hydrogenolysis using palladium on charcoal as catalyst at room temperature in a range of pressure between 0.05 and 0.3 MPa.

The amines of general formula (**III**) are subsequiently transformed to the corresponding ureas (**IV**) by reaction with the appropriate isocyanates of formula (**XIV**). This reaction is run in an inert solvent such as TH F or dichloromethane at a temperature between 10 and 40°C.

In an alternative way, compounds of formula (**IV**) can also be obtained by transforming the corresponding compounds of formula (**III**) into the corresponding isocyanates of formula (**IIIa**) by treatment of amines of formula (**III**) with a phosgene-like compound such as triphosgene in an inert solvent such as dichloromethane with the addition of a tertiary amine. Isocyanates of formula (**IIIa**) react with the appropriate amines R₄(CH₂)ₙNH2 in similar conditions to those described for the reaction of intermediates of formula (**III**) with intermediates of formula (**XIV**).

The ureas of formula (**IV**) thus obtained are deprotected to the corresponding amines of formula (**V**) according to the nature of the protecting group P₃. In the case of a benzyloxycarbonyl group, the deprotection is best carried through a hydrogenolysis using palladium on charcoal as catalyst at room temperature in a range of pressure between 0.05 and 0.3 MPa. If P₃ is a BOC group, the deprotection is best carried in acidic medium at room temperature using hydrogen chloride in dioxane or trifluoroacetic acid in dichloromethane as catalysts.

The reductive alkylation of the amines of formula (**V**) with the glyoxals of formula (**VI**) is carried out in a range of solvents or mixtures of solvents, for example methanol, THF or DMSO, in a range of temperatures from 0°C to 30°C and using a reducing agent such as sodium borohydride or sodium triacetoxy borohydride.

The amino alcohols of formula (**VII**) thus obtained are deprotected to the target compounds of general formula (**I**) by means of a hydrogenolysis in the case wherein P₁ is a benzyl group, using for example palladium on charcoal as catalyst in a solvent such as MeOH or mixtures with THF in neutral or slightly acidic conditions at room temperature and a hydrogen pressure between 0.1 and 0.3 MPa.

In the particular case wherein compounds of general formula (**I**) have chiral centres, they may be prepared following the sunthetic scheme depisted in Figure 2

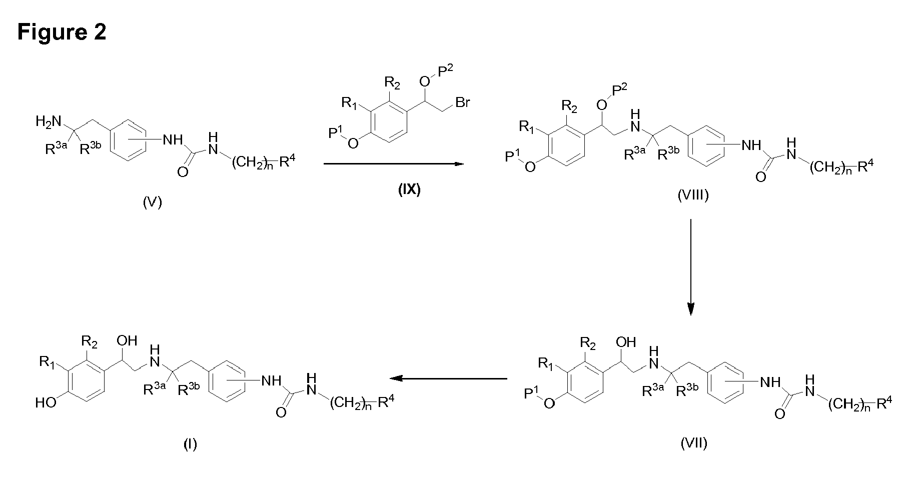

The already described amine derivative of formual (**V**) is alkylated with a protected bromohydrine of formula (**IX**) to give the intermediate of formula (**VIII**). This step is carried out in a range of temperatures between 80 and 140°C using a variety of solvents such as N-methylpirrolidone, DMSO, dioxane or toluene, and in the presence of an acid scavenger such as a tertiary amine or sodium bicarbonate.

The intermediate of formula (**VIII**) is deprotected to give intermediates of formula (**VII**), for example, in the presence of fluoride ion in THF when is used a trialkylsilyl protecting group as P². The resulting intermediate of formula (**VII**) is in turn deprotected to the target compound of formula (**I**) in a similar way to that described previously.

The following synthetic scheme depicted in figure 3 is envisaged in order to circumvent the low yields obtained in some cases for the alkylation of intermediates of general formula (V) (related to the thermal degradation of this intermediate).

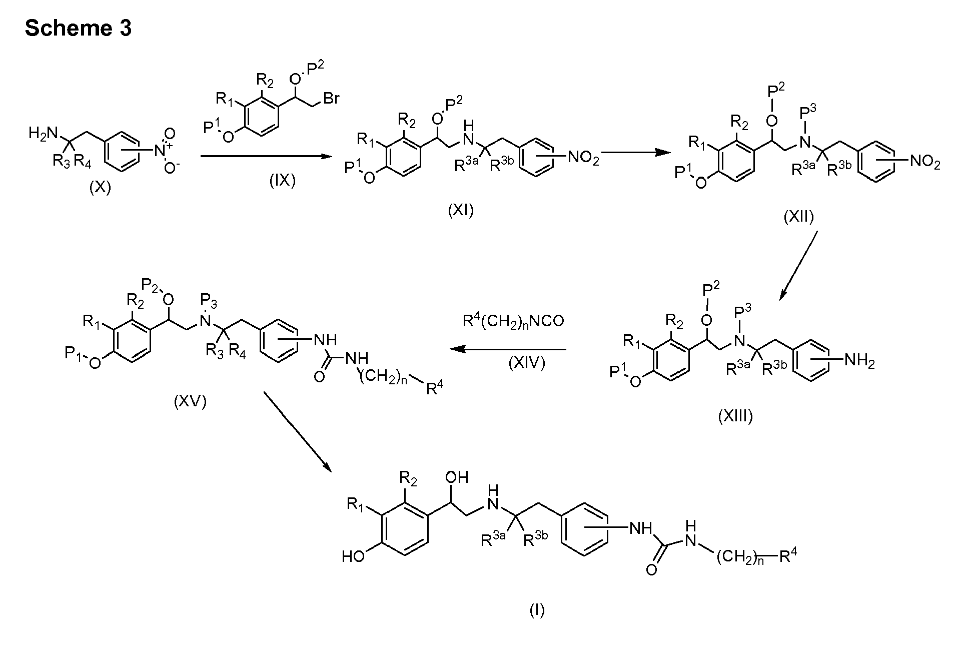

Here the known nitroamines of formula (**X**) are alkylated with the corresponding protected bromohydrines of formula (**IX**) in similar conditions to that described in Figure 2 for obtaining intermediates of formula (**VIII**) from intermediates (**V**) and intermediates (**IX**), being now the yields improved respect to the previous synthetic route depicted in Figure 2.

The Nitro derivatives of formula (**XI**) are then N-protected, for example by means of a BOC or a CBz group by treatment with the corresponding acylating agent in the presence of a basic catalyst as sodium hydroxide, potassium carbonate or triethylamine.

The next step involves the reduction of the nitro group of formula (**XII**) to the anilines of formula (**XIII**). This transformation is carried by hydrogenation using a catalyst such us Raney Nickel at room temperature in a range of pressures between 0.05 and 0.3 MPa.

Ureas derivatives of formula (**XV**) are readily prepared by treatment of anilines of formula (**XIII**) with an isocyanate of formula (**XIV**) in an inert solvent such us THF at room or somewhat higher temperature.

The transformation of intermediates of formula (**XV**) to the target compounds of fomrula (**I**) involves the successive deprotection of the groups P₁, P₂ and P₃. If P₃ is a BOC group, the deprotection is effected through an acidic treatment, usually hydrochloric or trifluoroacetic acid in an inert solvent at room temperature. If P₃ is a CBz group, the deprotection is best carried out by hydrogenolysis using Pd on charcoal as catalyst in similar conditions to that described previously.

In the particular case wherein R^{3a} is a methyl group, compounds of formula (**Ia**) are prepared according to scheme 4.

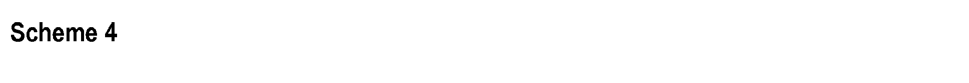

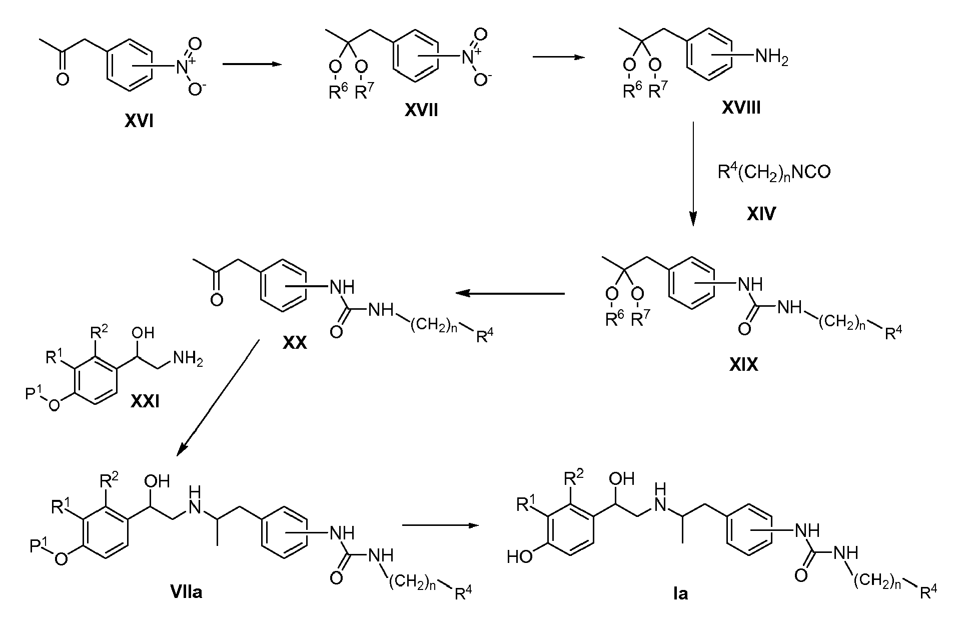

The known starting nitrophenylacetones of formula (**XVI**) are protected by means of an acyclic or cyclic ketal and thus transformed to intermediates of formula (**XVII**). This transformation is effected, for example, by treatment with a diol, such as ethyleneglycol, in the presence of an acid catalyst, such us p-toluenesulphonic acid, in an inert solvent with elimination of water.

The nitro derivatives of formula (**XVII**) are then transformed to the anilines of formula (**XVIII**) by conventional methods, for example by hydrogenation in the presence of a catalyst such us palladium on charcoal.

The corresponding ureas of formula (**XIX**) are obtained readily from intermediates of formula (**VIII**) by treatment, for example with an isocyanate of formula (**XIV**) in an inert solvent such as THF in a range of temperatures from 10°C to 40°C.

Intermediates of formula (**XIX**) are transformed into the phenylacetones of formula (**XX**) by treatment, for example, with aqueous hydrochloric acid in ethanol at a temperature between room temperature and 60°C.

In the next synthetic step the known aminoalcohols of formula (**XXI**) are reductively alkylated with the ketones of formula (**XX**). This step is carried out in the presence of a reductive agent such as sodium borohydride in a solvent like methanol or mixtures with THF or DMSO, usually at room temperature. The final deprotection of formula (**VIIa**) to compounds of formula (**Ia**) is effected in a similar way as described figure 1 for obtaining compounds of formula (**I**).

### EXAMPLES

**General.** Reagents, starting materials, and solvents were purchased from commercial suppliers and used as received. Concentration refers to evaporation under vacuum using a Büchi rotatory evaporator. Reaction products were purified, when necessary, by flash chromatography on silica gel (40-63 µm) with the solvent system indicated. Spectroscopic data were recorded on a Varian Gemini 300 spectrometer and a Varian Inova 400 spectrometer. HPLC-MS were performed on a Gilson instrument equipped with a Gilson piston pump 321, a Gilson 864 vacuum degasser, a Gilson liquid handler 215, a Gilson 189 injection module, a Gilson Valvemate 7000, a 1/1000 splitter, a Gilson 307 make-up pump, a Gilson 170 diode array detector, and a Thermoquest Finnigan aQa detector.

### Intermediate 1. 1-(3-nitrophenyl)acetone

To a solution of 3-nitrophenylacetic acid (5.4 g, 29.8 mmol) in pyridine (12.5 mL) was added anhydridous acetic acid (28 mL). The reaction mixture was stirred under nitrogen for 4h at room temperature. The solvent was removed under reduced pressure and the crude was treated with ethanol (30 mL) and concentrated hydrochloric acid (3 mL). The mixture was refluxed for 1 hour. The reaction crude was treated with ciclohexane and water and the organic layer was washed with water, sodium hydroxide 1 N, dried and the solvent was removed under reduced pressure. The title compound was obtained as a yellow solid (4 g, 75%) and used in the next step without further purification.

### Intermediate 2. 2-methyl-2-(3-nitrobenzyl)-1,3-dioxolane

To a solution of Intermediate 1 (1.5 g, 8.4 mmol) in toluene (15 mL) was added ethylene glycol (0.57 g, 8.4 mmol) and p-toluensulfonic acid (0.05 g, 0.29 mmol). The reaction mixture was stirred in a Dean-Stark for 4 hours. Hexane was added into the mixture and washed with water, sodium hydroxide 1 N and water. The organic layer was dried and the solvent was removed under reduced pressure giving the title compound as an oil (1.61 g, 86%), which was used in the next step without further purification.

### Intermediate 3. {3-[(2-methyl-1,3-dioxolan-2-yl)methyl]phenyl}amine

To a solution of Intermediate 2 (1.61 g, 7.2 mmol) in methanol (32 mL) was added palladium on charcoal (0.32 g, 10%). The reaction mixture was hydrogenated at 30 psi for 2 hours. The catalyst was filtered through Celite® and the solvent removed under reduced pressure obtaining the title compound as a solid (1.4 g, 99%), which was used in the next step without further purification.

### Intermediate 4. N-benzyl-N'-{3-[(2-methyl-1,3-dioxolan-2-yl)methyl]phenyl}urea

To a solution of Intermediate 3 (0.8 g, 4.1 mmol) in tetrahydrofuran (8 mL) was slowly added benzyl isocyanate (0.51 mL, 4.1 mmol). The reaction mixture was stirred at room temperature for 2 hours. The solvent was removed under reduced pressure obtaining an oil which crystallizes. The title compound was obtained as a solid (1.4 g, 99%).

### Intermediate 5. N-benzyl-N'-[3-(2-oxopropyl)phenyl]urea

To a solution of Intermediate 4 (1.4 g, 4.3 mmol) in acetic acid (14 mL) was added water (7 mL). The mixture was stirred at 80°C for 4 hours. The reaction crude was cooled to room temperature and water (28 mL) was slowly added. The precipitate was collected by filtration and washed with ether. The title compound was obtained as a solid (1.2 g, 99%).

### Intermediate 6. N-benzyl-N'-[3-((2R,S)-2-{[(2R,S)-2-(2,2-dimethyl-4H-1,3-benzodioxin-6-yl)-2-hydroxyethyl]amino}propyl)phenyl]urea

A solution of Intermediate 5 (0.6 g, 2.12 mmol) and 2-amino-1-(2,2-dimethyl-4H-benzo[d][1,3]dioxin-6-yl)ethanol (0.47 g, 2.12 mmol) in dimethylsulfoxide (7.3 mL) was stirred for 2 hours at room temperature. Then methanol (7.3 mL) was added into the reaction mixture and sodium borohydride (0.1 g, 2.3 mmol) was slowly added at 0°C. The reaction mixture was stirred at room temperature for 2 hours. The reaction crude was partitioned between ethyl acetate and water and the organic layer was washed several times with water, dried and the solvent was removed under reduced pressure. The title compound was obtained as an oil (1 g, 97%), which was used in the next step without further purification.

### EXAMPLE 1. N-benzyl-N'-{3-[(2R,S)-2-({(2R,S)-2-hydroxy-2-[4-hydroxy-3-(hydroxymethyl)phenyl]ethyl}amino)propyl]phenyl}urea

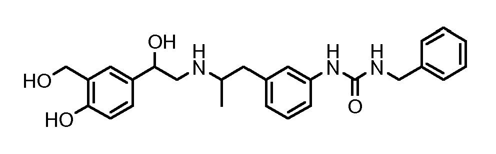

To a solution of Intermediate 6 (1 g, 2.04 mmol) in acetic acid (10 mL) was added water (5 mL). The reaction mixture was stirred at 80°C for 30 minutes. The solvent was removed under reduced pressure and the crude was partitioned between ethyl acetate and 1 N HCl. The aqueous phase was neutralized with sodium hydrogen carbonate until saturation, then the organic layer was extracted with a mixture of tetrahydrofuran and ethyl acetate and washed with water, dried and evaporated. The crude was obtained as an oil, which was treated with ether and the precipitate was collected by filtration giving the title compound as a solid (0.28 g, 63%). ¹H-NMR (300 MHz, dimethylsulfoxide-D6): 0.96 (d, *J*=6.41 Hz, 3H); 2.58-2.61 (m, 1H); 2.69-2.76 (m, 2H); 2.83-2.89 (m, 1H); 3.44 (bs, 1H); 4.34 (bs, 2H); 4.51 (s, 2H); 4.97 (bs, 1H); 6.62-6.65 (m, 1H); 6.71-6.78 (m, 2H); 7.01-7.04 (m, 1H); 7.13-7.19 (m, 1H); 7.27-7.43 (m, 7H); 8.54 (d, *J*=7.27 Hz, 1 H); 9.21 (bs, 1H). MS(M+): 450.

### Intermediate 7. 1-(4-nitrophenyl)acetone

Obtained from 4-nitropehnylacetic acid (5.4 g, 29.8 mmol), pyridine (12.5 mL) and anhydridous acetic acid (28 mL) by the same procedure described in Intermediate 1. The title compound was obtained as a yellow solid (4.5 g, 81 %) and used in the next step without further purification.

### Intermediate 8. 2-methyl-2-(4-nitrobenzyl)-1,3-dioxolane

Obtained from Intermediate 7 (1.5 g, 8.4 mmol), ethylene glycol (0.57 g, 8.4 mmol) and p-toluensulfonic acid (0.05 g, 0.29 mmol) by the same procedure described in Intermediate 2. The title compound was obtained as an oil (1.4 g, 75%) and used in the next step without further purification.

### Intermediate 9. {4-[(2-methyl-1,3-dioxolan-2-yl)methyl]phenyl}amine

Obtained from Intermediate 8 (1.3 g, 5.8 mmol) and palladium on charcoal (0.25 g, 10%) by the same procedure described in Intermediate 3. The title compound was obtained as a solid (0.9 g, 75%) and used in the next step without further purification.

### Intermediate 10. N-benzyl-N'-{4-[(2-methyl-1,3-dioxolan-2-yl)methyl]phenyl}urea

Obtained from Intermediate 9 (0.9 g, 4.6 mmol) and benzyl isocyanate (0.57 mL, 4.6 mmol) by the same procedure described in Intermediate 4. The title compound was obtained as white solid (1.5 g, 99%) and used in the next step without further purification.

### Intermediate 11. N-benzyl-N'-[4-(2-oxopropyl)phenyl]urea

Obtained from Intermediate 10 (1.5 g, 4.6 mmol), acetic acid (17 mL) and water (10 mL) by the same procedure described in Intermediate 5. The title compound was obtained as a yellow solid (1.3 g, 99%) and used in the next step without further purification.

### Intermediate 12. N-benzyl-N'-[4-((2R,S)-2-{[(2R,S)-(2,2-dimethyl-4H-1,3-benzodioxin-6-yl)-2-hydroxyethyl]amino}propyl)phenyl]urea

Obtained from Intermediate 11 (0.6 g, 2.12 mmol), 2-amino-1-(2,2-dimethyl-4H-benzo[d][1,3]dioxin-6-yl)ethanol (0.47 g, 2.12 mmol) and sodium borohydride (0.1 g, 2.3 mmol) by the same procedure described in Intermediate 6. The title compound was obtained as an oil (0.8 g, 77%) and used in the next step without further purification.

### EXAMPLE 2. N-benzyl-N'-{4-[(2R,S)-2-({(2R,S)-2-hydroxy-2-[4-hydroxy-3-(hydroxymethyl)phenyl]ethyl}amino)propyl]phenyl}urea

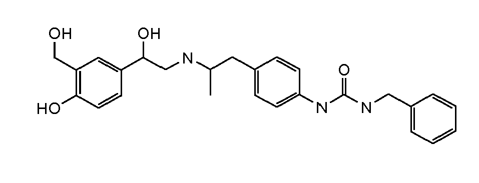

Obtained from Intermediate 12 (0.55 g, 1.12 mmol), acetic acid (5 mL) and water (2.5 mL) by the same procedure described in Example 1. The crude was treated with ether to give the title compound as a white solid (0.29 g, 63%). ¹H-NMR (300 MHz, dimethylsulfoxide-D6): 0.96 (d, *J*=6.04 Hz, 3H); 2.55-2.83 (m, 4H); 4.29 (d, *J*= 5.50 Hz, 2H); 4.46 (bs, 3H); 4.96 (bs, 1H); 6.58 (bs, 1H); 6.68 (d, *J*=8.24 Hz, 1H); 6.96-7.04 (m, 3H); 7.25-7.33 (m, 7H); 8.48 (s, 1H); 9.18 (bs, 1H). MS(M+): 450.

### Intermediate 13. N-benzyl-N'-{3-[(2R,S)-2-({(2R,S)-2-[8-(benzyloxy)-2-oxo-1,2-dihydroquinolin-5-yl]-2-hydroxyethyl}amino)propyl]phenyl}urea

Obtained from Intermediate 5 (0.4 g, 1.41 mmol), 5-(2-amino-1-hydroxyethyl)-8-(benzyloxy)quinolin-2(1 H)-one (0.44 g, 1.41 mmol) and sodium borohydride (0.1 g, 2.3 mmol) by the same procedure described in Intermediate 6. The title compound was obtained as a solid (0.072 g, 9%) and used in the next step without further purification.

### EXAMPLE 3. N-benzyl-N'-[3-((2R,S)-2-{[(2R,S)-2-hydroxy-2-(8-hydroxy-2-oxo-1,2-dihydroquinolin-5-yl)ethyl]amino}propyl)phenyl]urea

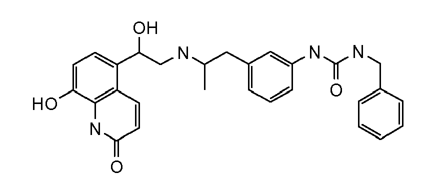

To a solution of Intermediate 13 (0.072 g, 0.125 mmol) in methanol (35 mL) was added palladium on charcoal (0.12 g, 10%). The reaction mixture was hydrogenated at 30 psi for 20 hours. The catalyst was filtered through Celite® and the solvent removed under reduced pressure. The crude obtained was treated with acetonitrile and ether to give the title compound as a solid (0.045 g, 70%). ¹H-NMR (300 MHz, dimethylsulfoxide-D6): 0.96 (d, *J*=6.4 Hz, 3H); 2.58-2.61 (m, 1H); 2.69-2.76 (m, 2H); 2.83-2.89 (m, 1H); 3.44 (bs, 1H); 4.51 (s, 2H); 5.3 (bs, 1H); 6.62 (d, *J*=9.89 Hz, 1H); 6.86 (bs, 1H); 7.05 (bs, 1H); 7.15-7.21 (m, 6H); 7.27-7.43 (m, 2H); 7.6 (m, 1H); 8.27 (d, *J*=9.89 Hz, 1H); 9.89 (bs, 1H). MS(M+): 487.

### Intermediate 14. N-(2-methoxybenzyl)-N'-{3-[(2-methyl-1,3-dioxolan-2-yl)methyl]phenyl}urea

Obtained from Intermediate 3 (0.55 g, 2.8 mmol) and 2-methoxybenzyl isocyanate (0.46 mL, 2.94 mmol) by the same procedure described in Intermediate 4. The title compound was obtained as an oil (0.99, 99%) and used in the next step without further purification.

### Intermediate 15. N-(2-methoxybenzyl)-N'-[3-(2-oxopropyl)phenyl]urea

Obtained from Intermediate 14 (1.3 g, 3.6 mmol), acetic acid (13 mL) and water (6.5 mL) by the same procedure described in Intermediate 5. The title compound was obtained as a brown solid (0.85 g, 75%) and used in the next step without further purification.

### Intermediate 16. N-[3-((2R,S)-2-{[(2R,S)-2-(2,2-dimethyl-4H-1,3-benzodioxin-6-yl)-2-hydroxyethyl]amino}propyl)phenyl]-N'-(2-methoxybenzyl)urea

Obtained from Intermediate 15 (0.45 g, 1.4 mmol), 2-amino-1-(2,2-dimethyl-4H-benzo[d][1,3]dioxin-6-yl)ethanol (0.32 g, 1.4 mmol) and sodium borohydride (0.06 g, 1.68 mmol) by the same procedure described in Intermediate 6. The title compound was obtained as a solid (0.76 g, 43%) and used in the next step without further purification.

### EXAMPLE 4. N-{3-[(2R,S)-2-({(2R,S)-2-hydroxy-2-[4-hydroxy-3-(hydroxymethyl)-phenyl]ethyl}amino)propyl]phenyl}-N'-(2-methoxybenzyl)urea

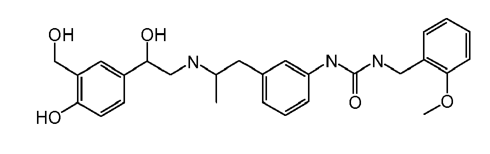

Obtained from Intermediate 16 (0.76 g, 1.46 mmol), acetic acid (7.6 mL) and water (3.8 mL) by the same procedure described in Example 1. The crude was purified by column chromatography with silica gel, eluting with methylene chloride/methanol/aqueous ammonia (40:4:0.2) to give the title compound as a solid (0.17 g, 54%). ¹H-NMR (300 MHz, dimethylsulfoxide-D6): 1.10 (d, *J*=6.04 Hz, 3H); 2.74-2.98 (m, 4H); 4.03 (s, 3H); 4.44 (bs, 1H); 4.65 (s, 2H); 5.18 (bs, 1H); 6.61 (bs, 1H); 6.85-6.92 (m, 2H); 7.09-7.20 (m, 3H); 7.26-7.33 (m, 1H); 7.39-7.46 (m, 4H); 8.73 (d, *J*=5.49 Hz, 1H); 9.37 (bs, 1H). MS(M+): 580.

### Intermediate 17. N-(2,6-dimethoxybenzyl)-N'-{3-[(2-methyl-1,3-dioxolan-2-yl)methyl]phenyl}urea

Obtained from Intermediate 3 (0.8 g, 4.1 mmol) and 2- (isocyanatemethyl)-1,3-dimethoxybenzene (0.8 g, 4.1 mmol) by the same procedure described in Intermediate 4. The title compound was obtained as a yellow solid (1.37 g, 85 %) and used in the next step without further purification.

### Intermediate 18. N-(2,6-dimethoxybenzyl)-N'-[3-(2-oxopropyl)phenyl]urea

Obtained from Intermediate 18 (1.4 g, 3.6 mmol), acetic acid (14 mL) and water (7 mL) by the same procedure described in Intermediate 5. The title compound was obtained (0.9 g, 69%) and used in the next step without further purification.

### Intermediate 19. N-(2,6-dimethoxybenzyl)-N'-[3-{(2R,S)-2-{[(2R)-(2,2-dimethyl-4H-1,3-benzodioxin-6-yl)-2-hydroxyethyl]amino}propyl)phenyl]urea

Obtained from Intermediate 18 (0.5 g, 1.46 mmol), (R)-2-amino-1-(2,2-dimethyl-4H-benzo[d][1,3]dioxin-6-yl)ethanol (0.33 g, 1.47 mmol), acetic acid (0.043 mL) and sodium borohydride (0.066 g, 1.75 mmol) by the same procedure described in Intermediate 6. The crude was obtained as an oil (0.73 g, 47%) and used in the next step without further purification.

### EXAMPLE 5. N-(2,6-dimethoxybenzyl)-N'-{3-[(2R,S)-2-({(2R)-2-hydroxy-2-[4-hydroxy-3-(hydroxymethyl)phenyl]ethyl}amino)propyl]phenyl}urea

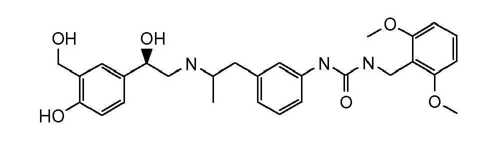

Obtained from Intermediate 19 (0.38 g, 0.69 mmol), acetic acid (4.3 mL) and water (3.65 mL) by the same procedure described in Example 1. The crude was purified by column chromatography with silica gel, eluting with methylene chloride/methanol/ammonia (40:8:1) and chloroform/methanol (95:5) to give the title compound as a solid (0.048 g, 12%). ¹H-NMR (300 MHz, dimethylsulfoxide-D6): 0.95 (bs, 6H); 2.39-2.85 (m, 4H); 4.17 (bs, 1H); 4.33 (s, 2H); 4.51 (s, 2H); 5.06 (bs, 1H); 6.09 (s, 1H); 6.72-6.74 (m, 4H); 7.03-7.3 (m, 6H); 8.47 (bs, 1H). MS(M+): 510.

### Intermediate 20. N-{3-[(2R,S)-2-({(2R,S)-2-[8-(benzyloxy)-2-oxo-1,2-dihydroquinolin-5-yl]-2-hydroxyethyl}amino)propyl]phenyl}-N'-(2-methoxybenzyl)urea

Obtained from Intermediate 15 (0.36 g, 1.15 mmol), 5-(2-amino-1-hydroxyethyl)-8-(benzyloxy)quinolin-2(1 H)-one (0.39 g, 1.15 mmol), sodium borohydride (0.06 g, 1.6 mmol) and acetic acid (0.06 mL) by the same procedure described in Intermediate 6. The crude was purified by column chromatography with silica gel, eluting with chloroform/methanol (95:5) to give the title compound as a foam (0.25 g, 34%).

### EXAMPLE 6. N-[3-((2R,S)-2-{[(2R,S)-2-hydroxy-2-(8-hydroxy-2-oxo-1,2-dihydroquinolin-5-yl)ethyl]amino}propyl)phenyl]-N'-(2-methoxybenzyl)urea

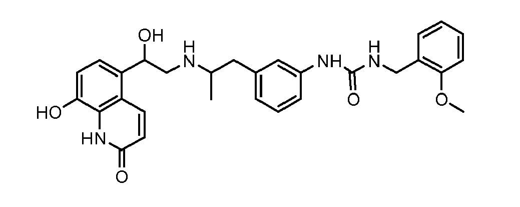

A solution of Intermediate 20 (0.25 g, 0.41 mmol) in methanol (25 mL) was added palladium on charcoal (0.05 g, 10%). The reaction mixture was hydrogenated under a balloon pressure for 20h. The catalyst was filtered through Celite® and the solvent removed under reduced pressure. The crude was treated with mehylen chloride and the precipitate was collected by filtration and washed with ethyl acetate to give the title compound as a yellow solid (0.183 g, 82%). ¹H-NMR (300 MHz, dimethylsulfoxide-D6): 1.09 (bs, 3H); 2.49-2.55 (m, 2H); 2.96 (bs, 3H); 3.88 (s, 3H); 4.3 (d, *J*=4.67 Hz, 2H); 5.24 (bs, 1H); 6.60 (d, *J*=7.97 Hz, 1H); 6.76 (t, *J*=6.87 Hz, 1H); 6.94-7.05 (m, 3H); 7.15-7.35 (m. 6H); 8.25 (bs, 1H); 8.75 (bs, 1H). MS(M+): 517.

### Intermediate 21. (3-nitrophenyl)acetonitrile

To a solution of 3-nitrobenzyl chloride (20.6 g, 0.12 mol) in methanol (120 mL) was added a solution of potassium cyanide (11 g, 0.15 mol) in water (20 mL). The reaction mixture was stirred at room temperature for 4 hours. The solvent was removed under reduced pressure and the crude was partitioned between ether and water. The organic layer was dried and evaporated. The crude was purified by column chromatography with silica gel, eluting with chloroform to give the title compound (11 g, 56%).

### Intermediate 22. [2-(3-nitrophenyl)ethyl]amine

Obtained from Intermediate 21 (10g, 0.061 mmol) and borane-methyl sulphide complex (40 mL, 0.42 mol) by the same procedure described in Journal of Medicinal Chemistry, 2003, 46, 9, p1668. The crude was treated with hydrogen chloride saturated with dioxane and the precipitate was collected by filtration and washed with ethyl acetate and ether. The title compound was obtained as a solid (4.9 g, 47%).

### Intermediate 23. tert-butyl [2-(3-nitrophenyl)ethyl]carbamate

To a solution of Intermediate 22 (4.9 g, 29.4 mmol) in tetrahydrofuran (77 mL) was added dropwise a solution of Boc anhydride (6.43 g, 29.4 mmol) in tetrahydrofuran (38 mL) at 0°C. The reaction mixture was stirred at room temperature for 2 hours. The solvent was removed under reduced pressure and the crude was partitioned between methylene chloride and water. The organic layer was washed several times with water, dried and evaporated. The title compound was obtained (7.5 g, 95%) and used in the next step without further purification.

### Intermediate 24. tert-butyl [2-(3-aminophenyl)ethyl]carbamate

To a solution of Intermediate 23 (7.5 g, 28.1 mmol) in methanol (300 mL) was added palladium on charcoal (1.5 g, 10%). The reaction mixture was hydrogenated under a balloon pressure for 3 hours at room temperature. The catalyst was filtered through Celite® and the solvent removed under reduced pressure obtaining the title compound (6 g, 90%), which was used in the next step without further purification.

### Intermediate 25. tert-butyl [2-(3-{[(benzylamino)carbonyl]amino}phenyl)ethyl]-carbamate

Obtained from Intermediate 24 (0.75 g, 2.8 mmol) and benzyl isocyanate (0.4 mL, 2.8 mmol) by the same procedure described in Intermediate 4. The crude was obtained as a solid (0.6 g, 50%) and used in the next step without further purification.

### Intermediate 26. N-[3-(2-aminoethyl)phenyl]-N'-benzylurea

To a solution of Intermediate 25 (0.6 g, 2.5 mmol) in dioxane (8 mL) was added acid chloride saturated with dioxane (8 mL). The reaction mixture was stirred for 2 hours at room temperature. The solvent was removed under reduced pressure and the crude was triturated with diisopropyl ether and the precipitate was collected by filtration giving the title compound as a solid (0.4 g, 47%), which was used in the next step without further purification.

### Intermediate 27. N-benzyl-N'-{3-[2-({(2R,S)-2-[4-(benzyloxy)-3-(hydroxymethyl)-phenyl]-2-hydroxyethyl}amino)ethyl]phenyl}urea

To a solution of Intermediate 26 (0.22 g, 0.8 mmol) and 1-(4-(benzyloxy)-3-(hydroxymethyl)phenyl)-2,2-dihydroxyethanone (0.2 g, 0.72 mmol) in a mixture of tetrhydrofuran/methanol 1:1 (6 mL) was added triethylamine (0.145 mL, 0.93 mmol). The mixture was stirred at room temperature for 2 hours. Then the mixture was cooled at 0°C and sodium borohydride (77 mg, 2.24 mmol) was slowly added into the reaction mixture, which was stirred for 2 hours at room temperature. The crude was partitioned between ether and water and the organic layer was washed several times with water, dried and evaporated. The crude was purified by column chromatography with silica gel, eluting with methylene chloride/methanol/ammonium (40:4:0.2) to obtain the title compound (0.32 g, 82%).

### EXAMPLE 7. N-benzyl-N'-{3-[2-({(2R,S)-2-hydroxy-2-[4-hydroxy-3-(hydroxymethyl)-phenyl]ethyl}amino)ethyl]phenyl}urea

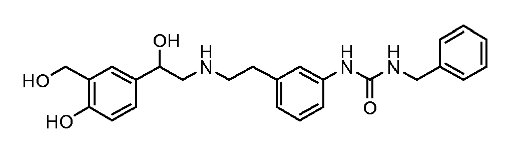

Obtained from Intermediate 27 (0.32 g, 0.6 mmol) and palladium on charcoal (0.064 g, 10%) by the same procedure described in Example 3. The crude was treated with ether to obtain the title compound as a solid (0.18 g, 68%). ¹H-NMR (300 MHz, dimethylsulfoxide-D6): 2.59-2.65 (m, 3H); 2.70-2.79 (m, 3H); 4.29 (d, *J*=5.87 Hz, 2H); 4.45 (s, 2H); 4.95 (bs, 1H); 6.60 (d, *J*=5.87 Hz, 1H); 6.68 (d, *J*=8.22 Hz, 1H); 6.74 (d, *J*=7.43 Hz, 1H); 6.97 (d, J=8.61 Hz, 1H); 7.11 (t, *J*=7.83 Hz, 1H); 7.22-7.35 (m, 7H); 8.52 (s, 1H). MS(M+): 436.

### Intermediate 28. N-benzyl-N'-{3-[2-({(2R,S)-2-[8-(benzyloxy)-2-oxo-1,2-dihydro-quinolin-5-yl]-2-hydroxyethyl}amino)ethyl]phenyl}urea

To a solution of Intermediate 26 (0.17 g, 0.63 mmol) and 8-(benzyloxy)-5-(2,2-dihydroxyacetyl)quinolin-2(1H)-one (0.23 g, 0.7 mmol) in a mixture of tetrahydrofuran/methanol 1:1 (5 mL) was added triethylamine (0.088 mL, 0.63 mmol). The reaction mixture was stirred at room temperature for 2 hours. Then the mixture was cooled at 0°C and sodium borohydride (0.08 mg, 2.1 mmol) was slowly added into the reaction mixture, which was stirred for 2 hours at room temperature.The crude was purified by column chromatography with silica gel, eluting with methylene chloride/methanol/ammonium (40:2.5:0.1) to give the title compound (0.24 g, 67%).

### EXAMPLE 8. N-benzyl-N'-[3-(2-{[(2R,S)-2-hydroxy-2-(8-hydroxy-2-oxo-1,2-dihydroquinolin-5-yl)ethyl]amino}ethyl)phenyl]urea

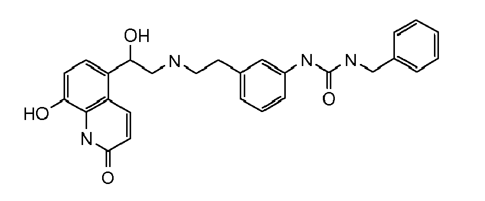

Obtained from Intermediate 28 (0.24 g, 0.42 mmol) and palladium on charcoal (0.06 g, 10%) by the same procedure described in Example 6 (reaction time: 15 hours). The crude was treated with ether to give the title compound as a solid (0.174 g, 80%). ¹H-NMR (300 MHz, dimethylsulfoxide-D6): 2.93-3.24 (m, 6H); 4.34 (d, *J*=5.77 Hz, 2H); 5.46 (bs, 1H); 6.24 (bs, 1H); 6.62 (d, *J*=9.89 Hz, 1H); 6.86 (dd, *J*=7.96, 8.5 Hz, 2H); 7.05 (d, *J*=8.24 Hz, 1H); 7.2-7.38 (m, 7H); 7.47 (s, 1H); 8.27 (d, *J*=9.89 Hz, 1H); 8.87 (s, 1H); 10.57 (bs, 1H). MS(M+): 473.

### Intermediate 29. tert-butyl {2-[3-({[(2-methoxybenzyl)amino]carbonyl}amino)phenyl]-ethyl}carbamate

Obtained from Intermediate 24 (0.8 g, 3.39 mmol) and 2-methoxyphenylisocyanate (0.58 g, 3.55 mmol) by the same procedure described in Intermediate 4. The crude was not isolated and used in the next step without further purification.

### Intermediate 30. N-[3-(2-aminoethyl)phenyl]-N'-(2-methoxybenzyl)urea

Obtained from Intermediate 29 and hydrogen chloride saturated dioxane (12 mL) by the same procedure described in Intermediate 26. The crude was treated with diisopropylether and the mixture was stirred for 30 minutes. The precipitate was collected by filtration and washed with ether and ethyl acetate to give the title compound as a solid (0.9 g, 46% global yield).

### Intermediate 31. N-{3-[2-({(2R,S)-[8-(benzyloxy)-2-oxo-1,2-dihydroquinolin-5-yl]-2-hydroxyethyl}amino)ethyl]phenyl}-N'-(2-methoxybenzyl)urea

Obtained from Intermediate 30 (0.21 g, 0.70 mmol), 8-(benzyloxy)-5-(2,2-dihydroxyacetyl)quinolin-2(1H)-one (0.24 g, 0.74 mmol) and sodium borohydride (0.078 g, 2.06 mmol) by the same procedure described in Intermediate 28. The crude was purified by column chromatography with silica gel, eluting with methylene chloride/methanol/ammonium (40:2.5:0.1) to give the title compound (0.28 g, 56%).

### EXAMPLE 9. N-[3-(2-{[(2R,S)-2-hydroxy-2-(8-hydroxy-2-oxo-1,2-dihydroquinolin-5-yl)ethyl]amino}ethyl)phenyl]-N'-(2-methoxybenzyl)urea

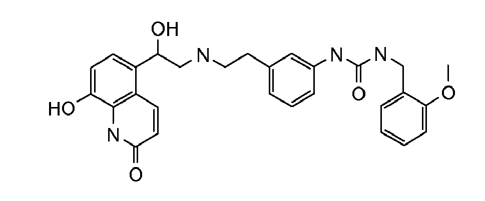

Obtained from Intermediate 31 (0.28 g, 0.47 mmol) and palladium on charcoal (0.056 g, 10%) by the same procedure described in Example 6 (reaction time: 48h). The crude was purified by column chromatography with silica gel, eluting with methylene chloride/methanol/ammonium (40:5:0.5) to give the title compound as a yellow solid (0.102 g, 42%). ¹H-NMR (300 MHz, dimethylsulfoxide-D6): 2.69-2.85 (m, 8H); 4.32 (s, 3H); 6.5 (bs, 1H); 6.57 (d, *J*=9.61 Hz, 1H); 6.79 (d, *J*=6.60 Hz, 1H); 6.96-7.00 (m, 2H); 7.05-7.20 (m, 3H); 7.26-7.32 (m, 4H); 8.23 (d, *J*=9.62 Hz, 1H); 8.61 (s, 1H). MS(M+): 503.

### Intermediate 32. tert-butyl [2-(3-isocyanatophenyl)ethyl]carbamate

To a solution of triphosgene (0.23 g, 0.78 mmol) in methylene chloride (2.6 mL) at 0°C was first added dropwise a solution of Intermediate 24 (0.5 g, 2.12 mmol) in methylene chloride (1 mL) and then a solution of trielthylamine (0.6 mL) in methylene chloride (1.7 mL). The reaction mixture was stirred at room temperature for 2.5 hours. The crude reaction was diluted with pentane (16 mL), which was added dropwise into the solution. A precipitate was filtered through Celite® and the filtrate gave the title compound, which was used in the next step as a solution.

### Intermediate 33. tert-butyl {2-[3-({[(4-fluorobenzyl)amino]carbonyl}amino)phenyl]-ethyl}carbamate

To a solution of Intermediate 32 (0.4 g, 1.5 mmol) was added 4-fluorobenzylamine (0.17 mL, 1.58 mmol) and the system was stirred overnight at room temperature. The solvent was removed under reduced pressure to give the title compound, which was used in the next step without further manipulation.

### Intermediate 34. N-[3-(2-aminoethyl)phenyl]-N'-(4-fluorobenzyl)urea

To a solution of Intermediate 33 in 4 mL of dioxane was added 8 mL of hydrogen chloride saturated dioxane. The reaction mixture was stirred at room temperature for 1 hour. The solvent was removed under reduced pressure and the crude was basified with potassium carbonate and extracted with ethyl acetate. The organic layer was dried and evaporated to give the title compound as an oil. The crude was purified by column chromatography with silica gel, eluting with methylene chloride/methanol/ammonium (from 40:2.5:0.1 to 40:8:1) to give the title compound (0.242 g, 56%).

### Intermediate 35. N-{3-[2-({(2R,S)-2-[8-(benzyloxy)-2-oxo-1,2-dihydroquinolin-5-yl]-2-hydroxyethyl}amino)ethyl]phenyl}-N'-(4-fluorobenzyl)urea

A solution of Intermediate 34 (0.24 g, 0.84 mmol) and 8-(benzyloxy)-5-(2,2-dihydroxyacetyl)quinolin-2(1H)-one (0.31 g, 0.95 mmol) in dimethyl sulfoxide (4 mL) was stirred at room temperature for 3 hours. Then methanol (4 mL) was added into the mixture and cooled at 0°C and sodium borohydride was slowly added. The reaction mixture was stirred at room temperature overnight. The crude reaction was diluted with water and the precipitate was collected by filtration. The crude was purified by column chromatography with silica gel, eluting with methylene chloride/methanol/ammonium (40:2.5:0.1) to give the title compound as a solid (0.3 g, 52%).

### EXAMPLE 10. N-(4-fluorobenzyl)-N'-[3-(2-{[(2R,S)-2-hydroxy-2-(8-hydroxy-2-oxo-1,2-dihydroquinolin-5-yl)ethyl]amino}ethyl)phenyl]urea

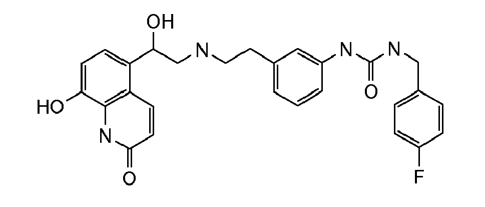

Obtained from Intermediate 35 (0.17 g, 0.23 mmol) and palladium on charcoal (0.017 g, 10%) by the same procedure described in Example 6 (reaction time: 4 hours). The crude was purified by column chromatography with silica gel, eluting with methylene chloride/methanol/ammonium (from 40:2.5:0.1 to 40:5:0.5) to give the title compound as a solid (0.07 g, 59%). ¹H-NMR (300 MHz, dimethylsulfoxide-D6): 2.6-3.26 (m, 6H); 4.26 (s, 2H); 4.96 (bs, 1H); 6.5 (d, *J*=9.75 Hz, 1H); 6.62 (bs, 1H); 6.73 (d, *J*=7.02 Hz, 1H); 6.91 (d, *J*=7.8 Hz, 1H); 7.04-7.37 (m. 7H); 8.17 (d, *J*=9.60 Hz, 1H); 8.52 (bs, 1H). MS(M+): 491.

### Intermediate 36. tert-butyl {2-[3-({[(2-phenylethyl)amino]carbonyl}amino)phenyl]-ethyl}carbamate

To a solution of Intermediate 32 (0.4 g, 1.5 mmol) was added phenethylamine (0.188 mL, 1.58 mmol) by the same procedure described in Intermediate 33. The solvent was removed under reduced pressure to give the title compound, which was used in the next step without further manipulation.

### Intermediate 37. N-[3-(2-aminoethyl)phenyl]-N'-(2-phenylethyl)urea

Obtained from Intermediate 36 and hydrogen chloride saturated dioxane by the same procedure described in Intermediate 34. The crude was purified by column chromatography with silica gel, eluting with methylene chloride/methanol/ammonium (from 40:2.5:0.1 to 40:8:1) to give the title compound (0.32 g, 75%).

### Intermediate 38. N-{3-[2-({(2R,S)-2-[8-(benzyloxy)-2-oxo-1,2-dihydroquinolin-5-yl]-2-hydroxyethyl}amino)ethyl]phenyl}-N'-(2-phenylethyl)urea

Obtained from Intermediate 37 (0.32 g, 1.13 mmol), 8-(benzyloxy)-5-(2,2-dihydroxyacetyl)quinolin-2(1H)-one (0.373 g, 1.15 mmol) and sodium borohydride (0.13 g, 3.44 mmol) by the same procedure described in Intermediate 35. The crude was purified by column chromatography with silica gel, eluting with methylene chloride/methanol/ammonium (40:2.5:0.1) to give the title compound as a solid (0.16 g, 23%).

### EXAMPLE 11. N-[3-(2-{[(2R,S)-2-hydroxy-2-(8-hydroxy-2-oxo-1,2-dihydroquinolin-5-yl)ethyl]amino}ethyl)phenyl]-N'-(2-phenylethyl)urea

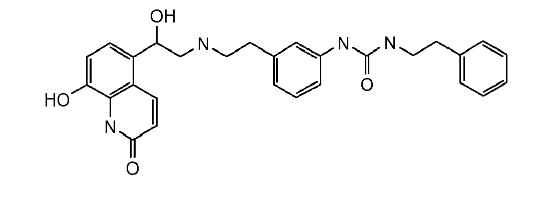

Obtained from Intermediate 38 (0.16 g, 0.28 mmol) and palladium on charcoal (0.032 g, 10%) by the same procedure described in Example 6. The crude obtained was purified by column chromatography with silica gel, eluting with chloroform/methanol (from 15:1 to 8:1) to give the title compound as a solid (0.085 g, 62%). ¹H-NMR (300 MHz, dimethylsulfoxide-D6): 1.07-1.27 (m, 2H); 2.71-2.93 (m, 8H); 5.13 (bs, 1H); 6.16 (bs, 1H); 6.53 (d, *J*=9.7 Hz, 1H); 6.74 (bs, 1H); 6.94 (bs, 1H); 7.09-7.31 (m, 8H); 8.18 (d, *J*=9.62 Hz, 1H); 8.50 (bs, 1H). MS(M+): 487.

### Intermediate 39. tert-butyl {2-[3-({[(3-phenylpropyl)amino]carbonyl}amino)phenyl]-ethyl}carbamate

Obtained from Intermediate 32 (0.4 g, 1.5 mmol) and 3-phenylpropilamine (0.213 mL, 1.58 mmol) by the same procedure described in Intermediate 33. The solvent was removed under reduced pressure to give the title compound, which was used in the next step without further manipulation.

### Intermediate 40. N-[3-(2-aminoethyl)phenyl]-N'-(3-phenylpropyl)urea

Obtained from Intermediate 39 and hydrogen chloride saturated dioxane by the same procedure described in Intermediate 34. The crude was purified by column chromatography with silica gel, eluting with methylene chloride/methanol/ammonium (from 40:2.5:0.1 to 40:8:1) to give the title compound (0.21 g, 47%).

### Intermediate 41. N-{3-[2-({(2R,S)-2-[8-(benzyloxy)-2-oxo-1,2-dihydroquinolin-5-yl]-2-hydroxyethyl}amino)ethyl]phenyl}-N'-(3-phenylpropyl)urea

Obtained from Intermediate 40 (0.21 g, 0.71 mmol), 8-(benzyloxy)-5-(2,2-dihydroxyacetyl)quinolin-2(1H)-one (0.253 g, 0.78 mmol) and sodium borohydride (0.087 g, 2.3 mmol) by the same procedure described in Intermediate 35. The crude was purified by column chromatography with silica gel, eluting with chloroform/methanol (from 50:1 to 25:1) to give the title compound as a solid (0.193 g, 46%).

### EXAMPLE 12. N-[3-(2-{[(2R,S)-2-hydroxy-2-(8-hydroxy-2-oxo-1,2-dihydroquinolin-5-yl)ethyl]amino}ethyl)phenyl]-N'-(3-phenylpropyl)urea

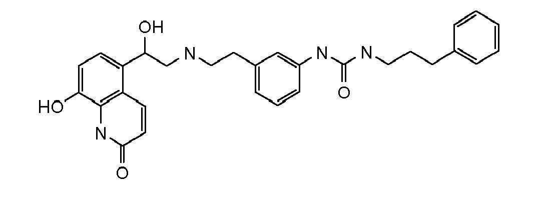

Obtained from Intermediate 41 (0.19 g, 0.33 mmol) and palladium on charcoal (0.038 g, 10%) by the same procedure described in Example 6. The crude was treated with ether obtaining the title compound as a solid (0.064 g, 38 %). ¹H-NMR (300 MHz, dimethylsulfoxide-D6): 1.03-1.10 (m, 4H); 2.86-3.22 (m, 8H); 5.38 (bs, 1H); 6.16 (bs, 1H); 6.35 (bs, 1H); 6.59 (bs, 1H); 6.78 (bs, 1H); 7.01 (bs, 1H); 7.18-7.43 (m, 7H); 8.22 (bs, 1H). MS(M+): 501.

### Intermediate 42. N-{3-[2-({(2R,S)-2-[4-(benzyloxy)-3-(hydroxymethyl)phenyl]-2-hydroxyethyl}amino)ethyl]phenyl}-N'-(3-phenylpropyl)urea

Obtained from Intermediate 40 (0.33 g, 1.11 mmol), 1-(4-(benzyloxy)-3-(hydroxymethyl)phenyl)-2,2-dihydroxyethanone (0.30 g, 1.11 mmol) and sodium borohydride (0.2 g, 4.49 mmol) by the same procedure described in Intermediate 35. The crude was purified by column chromatography with silica gel, eluting with chloroform/methanol (90:5) to give the title compound as an oil (0.18 g, 29%).

### EXAMPLE 13. N-{3-[2-({(2R,S)-2-hydroxy-2-[4-hydroxy-3-(hydroxymethyl)-phenyl]ethyl}amino)ethyl]phenyl}-N'-(3-phenylpropyl)urea

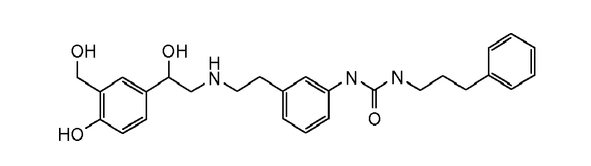

Obtained from Intermediate 42 (0.18 g, 0.33 mmol) and palladium on charcoal (0.05 g, 10%) by the same procedure described in Example 3 (pressure: 40psi, reaction time: 3 hours). The crude was treated with ether to give the title compound as a solid (0.09 g, 60%). ¹H-NMR (300 MHz, methanol): 0.89 (bs, 2H); 1.34 (bs, 2H); 2.66-2.90 (m, 7H); 3.22 (bs, 1H); 4.69 (bs, 1H); 6.71-6.84 (m, 3H); 7.06-7.27 (m, 9H). MS(M+): 464.

### Intermediate 43. tert-butyl {2-[3-({[(diphenylmethyl)amino]carbonyl}amino)phenyl]-ethyl}carbamate

Obtained from Intermediate 32 (0.4 g, 1.5 mmol) and 1,1-diphenylmethylamine (0.259 mL, 1.58 mmol) by the same procedure described in Intermediate 33. The solvent was removed under reduced pressure to give the title compound, which was used in the next step without further manipulation.

### Intermediate 44. N-[3-(2-aminoethyl)phenyl]-N'-(diphenylmethyl)urea

Obtained from Intermediate 43 and hydrogen chloride saturated dioxane by the same procedure described in Intermediate 34. The crude was purified by column chromatography with silica gel, eluting with methylene chloride/methanol/ammonium (from 40:2.5:0.1 to 40:8:1) to give the title compound (0.24 g, 46%).

### Intermediate 45. N-{3-[2-({(2R,S)-2-[8-(benzyloxy)-2-oxo-1,2-dihydroquinolin-5-yl]-2-hydroxyethyl}amino)ethyl]phenyl}-N'-(diphenylmethyl)urea

Obtained from Intermediate 44 (0.24 g, 0.69 mmol), 8-(benzyloxy)-5-(2,2-dihydroxyacetyl)quinolin-2(1 H)-one (0.233 g, 0.72 mmol) and sodium borohydride (0.082 g, 2.17 mmol) by the same procedure described in Intermediate 35. The crude was purified by column chromatography with silica gel, eluting with chloroform/methanol (from 50:1 to 30:1) to give the title compound as a solid (0.33 g, 72%).

### EXAMPLE 14. N-(diphenylmethyl)-N'-[3-(2-{[(2R,S)-2-hydroxy-2-(8-hydroxy-2-oxo-1,2-dihydroquinolin-5-yl)ethyl]amino}ethyl)phenyl]urea

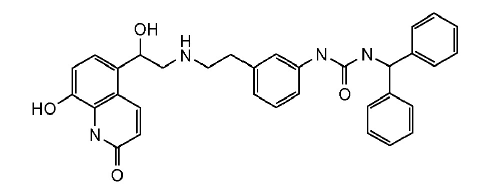

Obtained from Intermediate 45 (0.33 g, 0.52 mmol) and palladium on charcoal (0.06 g, 10%) by the same procedure described in Example 6 (reaction time: overnight). The crude was treated with ether obtaining the title compound as a solid (0.2 g, 69 %). ¹H-NMR (300 MHz, dimethylsulfoxide-D6): 2.88-2.95 (m, 2H); 3.05-3.16 (m, 4H); 5.39 (bs, 1H); 5.95 (d, *J*=8.24 Hz, 1H); 6.17 (bs, 1H); 6.57 (d, *J*=9.89 Hz, 1H); 6.78 (bs, 1H); 7.00 (d, *J*=7.97 Hz, 1H); 7.14-7.26 (m, 4H); 7.31-7.40 (m, 10H); 7.48 (d, *J*=8.24 Hz, 1H); 8.21 (d, *J*= 10.116 Hz, 1H); 8.86 (bs, 1H). MS(M+): 549.

### Intermediate 46. tert-butyl (2-{3-[(anilinocarbonyl)amino]phenyl}ethyl)carbamate

Obtained from Intermediate 32 (0.4 g, 1.5 mmol) and aniline (0.137 mL, 1.575 mmol) by the same procedure described in Intermediate 33. The solvent was removed under reduced pressure to give the title compound, which was used in the next step without further manipulation.

### Intermediate 47. N-[3-(2-aminoethyl)phenyl]-N'-phenylurea

Obtained from Intermediate 46 and hydrogen chloride saturated dioxane by the same procedure described in Intermediate 34. The crude was purified by column chromatography with silica gel, eluting with methylene chloride/methanol/ammonium (from 40:2.5:0.1 to 40:8:1) to give the title compound (0.2 g, 52%).

### Intermediate 48. N-{3-[2-({(2R,S)-[8-(benzyloxy)-2-oxo-1,2-dihydroquinolin-5-yl]-2-hydroxyethyl}amino)ethyl]phenyl}-N'-phenylurea

Obtained from Intermediate 47 (0.2 g, 0.78 mmol), 8-(benzyloxy)-5-(2,2-dihydroxyacetyl)quinolin-2(1H)-one (0.27 g, 0.83 mmol) and sodium borohydride (0.088 g, 2.32 mmol) by the same procedure described in Intermediate 35. The crude obtained was purified by column chromatography with silica gel, eluting with chloroform/methanol (4:1) to give the title compound as a solid (0.24 g, 47%).

### EXAMPLE 15. N-[3-(2-{[(2R,S)-2-hydroxy-2-(8-hydroxy-2-oxo-1,2-dihydroquinolin-5-yl)ethyl]amino}ethyl)phenyl]-N'-phenylurea

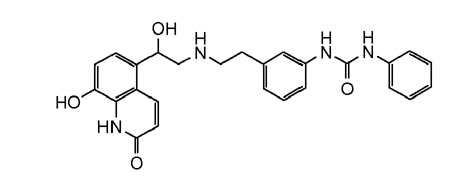

Obtained from Intermediate 48 (0.24 g, 0.44 mmol) and palladium on charcoal (0.048 g, 10%) by the same procedure described in Example 6 (reaction time: 48 hours). The crude was purified by column chromatography with silica gel, eluting with chloroform/methanol (6:1). The title compound was obtained as a yellow solid (0.067 g, 32%). ¹H-NMR (300 MHz, dimethylsulfoxide-D6): 2.92-3.18 (m, 4H); 5.37 (bs, 1H); 6.63 (d, *J*=9.71 Hz, 1H); 6.9 (d, *J*=6.87 Hz, 1H); 7.03 (m, 2H); 7.19-7.35 (m, 5H); 7.51 (bs, 3H); 8.26 (d, *J*=9.88 Hz, 1H); 9.00 (bs, 1H). MS(M+): 459.

### Intermediate 49. tert-butyl [2-(3-{[(1-adamantylamino)carbonyl]amino}phenyl)-ethyl]carbamate

To a solution of Intermediate 24 (0.3 g, 1.27 mmol) in tetrahydrofuran (3 mL) was slowly added 1-isocyanate-adamantane (0.23 g, 1.3 mmol). The reaction mixture was stirred under Argon at room temperature overnight and for 4 hours at 92°C. The solvent was removed under reduced pressure and the title compound was obtained as a solid (0.52 g, 94%) and used in the next step without further purification.

### Intermediate 50. N-1-adamantyl-N'-[3-(2-aminoethyl)phenyl]urea

Obtained from Intermediate 49 (0.52 g, 1.26 mmol) and hydrogen chloride saturated dioxane (2.4 mL) by the same procedure described in Intermediate 34 (reaction time: 3 hours). The crude was obtained as a foam (0.219 g, 54%) and used in the next step without further purification.

### Intermediate 51. N-1-adamantyl-N'-{3-[2-({(2R,S)-2-[8-(benzyloxy)-2-oxo-1,2-dihydroquinolin-5-yl]-2-hydroxyethyl}amino)ethyl]phenyl}urea

Obtained from Intermediate 50 (0.21 g, 0.67 mmol), 8-(benzyloxy)-5-(2,2-dihydroxyacetyl)quinolin-2(1H)-one (0.22 g, 0.68 mmol) and sodium borohydride (0.077 g, 2.04 mmol) by the same procedure described in Intermediate 35. The title compound was obtained as a solid (0.35 g, 81 %) and used in the next step without further purification.

### EXAMPLE 16. N-1-adamantyl-N'-[3-(2-{[(2R,S)-2-hydroxy-2-(8-hydroxy-2-oxo-1,2-dihydroquinolin-5-yl)ethyl]amino}ethyl)phenyl]urea

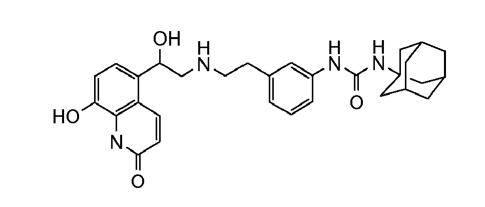

Obtained from Intermediate 51 (0.35 g, 0.58 mmol) and palladium on charcoal (0.07 g, 10%) by the same procedure described in Example 6 (reaction time: 72 hours). The crude was treated with ether and purified by column chromatography with silica gel, eluting with chloroform/methanol (form 10:1 to 6:1) to give the title compound as a solid (0.138 g, 44%). ¹H-NMR (300 MHz, dimethylsulfoxide-D6): 1.64 (bs, 5H); 1.93 (bs, 7H); 2.02 (bs, 3H); 2.61-2.81 (m, 6H); 5.03 (bs, 1H); 5.86 (s, 1H); 6.51 (d, *J*=9.62 Hz, 1H); 6.70 (bs, 1H); 6.91 (d, *J*=8.24 Hz, 1H); 7.08 (bs, 2H); 7.27 (bs, 1H); 8.17 (d, *J*=9.06 Hz, 1H). MS(M+): 517.

### Intermediate 52. tert-butyl {2-[3-({[(2,6-dichlorobenzyl)amino]carbonyl}amino)-phenyl]ethyl}carbamate

Obtained from Intermediate 32 (0.68 g, 2.6 mmol) and (2,6-dichlorophenyl)methanamine (0.503 mL, 2.86 mmol) by the same procedure described in Intermediate 33. The solvent was removed under reduced pressure to give the title compound, which was used in the next step without further manipulation.

### Intermediate 53. N-[3-(2-aminoethyl)phenyl]-N'-(2,6-dichlorobenzyl)urea

Obtained from Intermediate 52 and hydrogen chloride saturated dioxane by the same procedure described in Intermediate 34. The crude was purified by column chromatography with silica gel, eluting with methylene chloride/methanol/ammonium (from 40:2.5:0.1 to 40:4:2) to give the title compound (0.416 g, 47%).

### Intermediate 54. N-{3-[2-({(2R,S)-2-[8-(benzyloxy)-2-oxo-1,2-dihydroquinolin-5-yl]-2-hydroxyethyl}amino)ethyl]phenyl}-N'-(2,6-dichlorobenzyl)urea

Obtained from Intermediate 53 (0.41 g, 1.23 mmol), 8-(benzyloxy)-5-(2,2-dihydroxyacetyl)quinolin-2(1H)-one (0.36 g, 1.11 mmol) and sodium borohydride (0.139 g, 3.67 mmol) by the same procedure described in Intermediate 35. The crude was purified by column chromatography in reverse phase, eluting with acetonitrile/water (1:1) to give the title compound as a solid (0.193 g, 27%).

### EXAMPLE 17. N-(2,6-dichlorobenzyl)-N'-[3-(2-{[(2R,S)-2-hydroxy-2-(8-hydroxy-2-oxo-1,2-dihydroquinolin-5-yl)ethyl]amino}ethyl)phenyl]urea

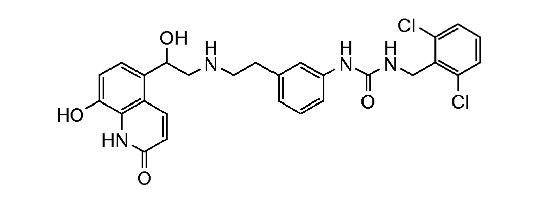

Obtained from Intermediate 54 (0.19 g, 0.3 mmol) and palladium on charcoal (0.02 g, 10%) by the same procedure described in Example 6 (reaction time: overnight). The crude was purified by column chromatography with silica gel, eluting with chloroform/methanol (4:1) and the title compound was obtained as a solid (0.041 g, 25%). ¹H-NMR (300 MHz, dimethylsulfoxide-D6): 1.58-1.87 (m, 2H); 1.96-2.17 (m, 2H); 2.40-2.47 (m, 2H); 3.45 (s, 2H); 4.76-4.9 (m, 1H); 6.88 (bs, 1H); 7.19 (bs, 1H); 7.29-7.43 (m, 5H); 7.49-7.54 (m, 3H); 8.25 (bs, 1H); 9.61 (bs, 1H). MS(M+): 542.

### Intermediate 55. tert-butyl [2-(3-{[(biphenyl-2-ylamino)carbonyl]amino}phenyl)ethyl]-carbamate

Obtained from Intermediate 32 (0.68 g, 2.6 mmol) and biphenyl-2-amine (0.483 g, 2.86 mmol) by the same procedure described in Intermediate 33. The solvent was removed under reduced pressure to give the title compound, which was used in the next step without further manipulation.

### Intermediate 56. N-[3-(2-aminoethyl)phenyl]-N'-biphenyl-2-ylurea

Obtained from Intermediate 55 and hydrogen chloride saturated dioxane by the same procedure described in Intermediate 34. The crude was purified by column chromatography with silica gel, eluting with methylene chloride/methanol/ammonium (40:2.5:0.1) to give the title compound (0.513 g, 59%).

### Intermediate 57. N-{3-[2-({(2R,S)-2-[8-(benzyloxy)-2-oxo-1,2-dihydroquinolin-5-yl]-2-hydroxyethyl}amino)ethyl]phenyl}-N'-biphenyl-2-ylurea

Obtained from Intermediate 56 (0.513 g, 1.55 mmol), 8-(benzyloxy)-5-(2,2-dihydroxyacetyl)quinolin-2(1H)-one (0.503 g, 1.55 mmol) and sodium borohydride (0.175 g, 4.63 mmol) by the same procedure described in Intermediate 35. The crude was purified by column chromatography with silica gel, eluting with chloroform/methanol (25:1) to give the title compound as a yellow foam (0.759 g, 76%).

### EXAMPLE 18. N-biphenyl-2-yl-N'-[3-(2-{[(2RS)-2-hydroxy-2-(8-hydroxy-2-oxo-1,2-dihydroquinolin-5-yl)ethyl]amino}ethyl)phenyl]urea

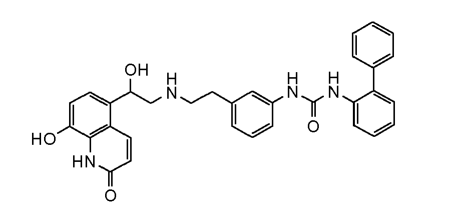

Obtained from Intermediate 57 (0.56 g, 0.9 mmol) and palladium on charcoal (0.05 g, 10%) by the same procedure described in Example 6 (reaction time: 32 hours). The crude was purified by column chromatography with silica gel, eluting with chloroform/methanol (4:1) and the title compound was obtained as a solid (0.258 g, 52%). ¹H-NMR (300 MHz, dimethylsulfoxide-D6): 2.92-3.24 (m, 6H); 5.41 (bs, 1H); 6.63 (d, *J*=9.61 Hz, 1H); 6.89 (bs, 1H);7.04 (d, *J*=8.24 Hz, 1H); 7.17-7.29 (m, 4H); 7.36-7.50 (m, 5H); 7.53-7.58 (m, 2H); 7.77 (s, 1H); 7.96 (d, *J*=8.24 Hz, 1H); 8.27 (d, *J*=9.61 Hz, 1H); 9.15 (bs, 1H). MS(M+): 535.

### Intermediate 58. tert-butyl [2-(3-{[(cyclohexylamino)carbonyl]amino}phenyl)ethyl]-carbamate

Obtained from Intermediate 32 (0.708 g, 2.7 mmol) and cyclohexanamine (0.56 g, 5.6 mmol) by the same procedure described in Intermediate 33. The solvent was removed under reduced pressure to give the title compound, which was used in the next step without further manipulation.

### Intermediate 59. N-[3-(2-aminoethyl)phenyl]-N'-cyclohexylurea

Obtained from Intermediate 58 and hydrogen chloride saturated dioxane by the same procedure described in Intermediate 34. The crude was treated with acetonitrile, isopropylether and ether to give a precipitate, which was collected by filtration. The title compound was obtained as a solid (0.3 g, 42%) and used in the next step without further purification.

### Intermediate 60. N-{3-[2-({(2R,S)-[8-(benzyloxy)-2-oxo-1,2-dihydroquinolin-5-yl]-2-hydroxyethyl}amino)ethyl]phenyl}-N'-cyclohexylurea

A solution of Intermediate 59 (0.3 g, 1.15 mmol) and 8-(benzyloxy)-5-(2,2-dihydroxyacetyl)quinolin-2(1H)-one (0.4 g, 1.23 mmol) in a mixture of tetrahydrofuran/methanol 1:1 (7 mL) was stirred for 3 hours at room temperature. The mixture was cooled at 0°C and sodium borohydride (0.12 g, 3.17 mmol) was slowly added. The reaction was stirred at room temperature overnight. The crude reaction was diluted with ethyl acetate and washed with water. The solvent was removed under reduced pressure and the crude was purified by column chromatography with silica gel, eluting with clhorofom/methanol (from 1:0 to 8:1) to give the title compound (0.3 g, 43%).

### EXAMPLE 19. N-cyclohexyl-N'-[3-(2-{[(2R,S)-2-hydroxy-2-(8-hydroxy-2-oxo-1,2-dihydroquinolin-5-yl)ethyl]amino}ethyl)phenyl]urea

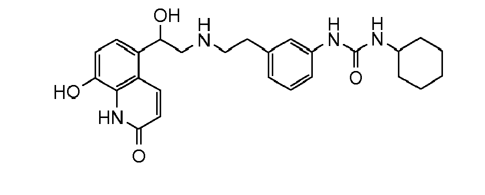

Obtained from Intermediate 60 (0.3 g, 0.54 mmol) and palladium on charcoal (0.075 g, 10%) by the same procedure described in Example 6 (reaction time: 48 hours). The crude was purified by column chromatography in reverse phase, eluting with acetonitrile/water to give the title compound as a solid (0.08 g, 32%). ¹H-NMR (300 MHz, dimethylsulfoxide-D6): 1.14-1.40 (m, 5H); 1.53-1.86 (m, 5H); 2.75-3.03 (m, 7H); 5.23 (bs, 1H); 6.33 (d, *J*=7.24 Hz, 1H); 6.58 (d, *J*=9.88 Hz, 1H); 6.78 (d, *J*=7.69 Hz, 1H); 7.00 (d, *J*=8.24 Hz, 1H); 7.14-7.19 (m, 2H); 7.24 (d, *J*=7.96 Hz, 1H); 7.35 (s, 1H); 8.23 (d, *J*=9.89 Hz, 1H); 8.41 (s, 1H); 8.53 (s, 1H). MS(M+): 465.

### Intermediate 61. tert-butyl {2-[3-({[(4-phenylbutyl)amino]carbonyl}amino)phenyl]-ethyl}carbamate

Obtained from Intermediate 32 (0.708 g, 2.7 mmol) and 4-phenylbutan-1-amine (0.42 g, 2.97 mmol) by the same procedure described in Intermediate 33. The solvent was removed under reduced pressure to give the title compound, which was used in the next step without further manipulation.

### Intermediate 62. N-[3-(2-aminoethyl)phenyl]-N'-(4-phenylbutyl)urea.

Obtained from Intermediate 61 and hydrogen chloride saturated dioxane by the same procedure described in Intermediate 34. The crude was treated with acetonitrile, isopropylether and ether to give a precipitate, which was collected by filtration. The title compound was obtained as a solid (0.36 g, 43%) and used in the next step without further purification.

### Intermediate 63. N-{3-[2-({(2R,S)-2-[8-(benzyloxy)-2-oxo-1,2-dihydroquinolin-5-yl]-2-hydroxyethyl}amino)ethyl]phenyl}-N'-(4-phenylbutyl)urea

Obtained from Intermediate 62 (0.36 g, 1.16 mmol), 8-(benzyloxy)-5-(2,2-dihydroxyacetyl)quinolin-2(1H)-one (0.4 g, 1.23 mmol) and sodium borohydride (0.1 g, 3.2 mmol) by the same procedure described in Intermediate 35. The crude was purified by column chromatography with silica gel, eluting with clhorofom/methanol (from 1:0 to 8:1) to give the title compound (0.44 g, 59%).

### EXAMPLE 20. N-[3-(2-{[(2R,S)-2-hydroxy-2-(8-hydroxy-2-oxo-1,2-dihydroquinolin-5-yl)ethyl]amino}ethyl)phenyl]-N'-(4-phenylbutyl)urea

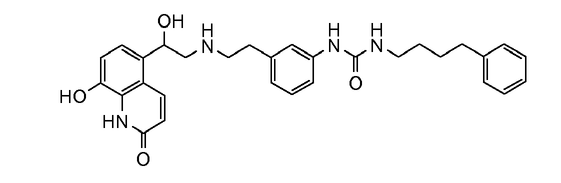

Obtained from Intermediate 63 (0.4 g, 0.66 mmol) and palladium on charcoal (0.1 g, 10%) by the same procedure described in Example 6 (reaction time: 48h). The crude was purified by column chromatography in reverse phase, eluting with acetonitrile/water to obtain the title compound as a solid (0.085 g, 25%). ¹H-NMR (300 MHz, dimethylsulfoxide-D6): 1.42-1.52 (m, 2H); 1.57-1.67 (m, 2H); 2.59-2.65 (m, 2H); 2.74-2.79 (m, 2H); 2.86-2.97 (m, 4H); 3.09-3.16 (m, 2H); 5.18 (bs, 1H); 6.55 (d, *J*=9.89 Hz, 1H); 6.60 (bs, 1H); 6.75 (d, *J*=7.15 Hz, 1H); 6.99 (d, *J*=8.24 Hz, 1H); 7.13 (d, *J*=9.06 Hz, 1H); 7.17-7.33 (m, 7H); 8.22 (d, *J*=9.89 Hz, 1H); 8.79 (bs, 1H). MS(M+): 515.

### Intermediate 64. tert-butyl {2-[3-({[(4-methoxyphenyl)amino]carbonyl}amino)-phenyl]ethyl}carbamate

Obtained from Intermediate 32 (0.708 g, 2.7 mmol) and 4-methoxyaniline (0.34 g, 2.97 mmol) by the same procedure described in Intermediate 33. The solvent was removed under reduced pressure to give the title compound, which was used in the next step without further manipulation.

### Intermediate 65. N-[3-(2-aminoethyl)phenyl]-N'-(4-methoxyphenyl)urea

Obtained from Intermediate 64 and hydrogen chloride saturated dioxane by the same procedure described in Intermediate 34. The crude was treated with acetonitrile, isopropylether and ether to give a precipitate, which was collected by filtration. The title compound was obtained as a solid (0.33 g, 43%) and used in the next step without further purification.

### Intermediate 66. N-{3-[2-({(2R,S)-2-[8-(benzyloxy)-2-oxo-1,2-dihydroquinolin-5-yl]-2-hydroxyethyl}amino)ethyl]phenyl}-N'-(4-methoxyphenyl)urea

Obtained from Intermediate 65 (0.33 g, 1.16 mmol), 8-(benzyloxy)-5-(2,2-dihydroxyacetyl)quinolin-2(1H)-one (0.4 g, 1.23 mmol) and sodium borohydride (0.12 g, 3.2 mmol) by the same procedure described in Intermediate 35. The crude was purified by column chromatography with silica gel, eluting with chlorofom/methanol (from 1:0 to 8:1) to give the title compound (0.4 g, 58%).

### EXAMPLE 21. N-[3-(2-{[(2R,S)-2-hydroxy-2-(8-hydroxy-2-oxo-1,2-dihydroquinolin-5-yl)ethyl]amino}ethyl)phenyl]-N'-(4-methoxyphenyl)urea

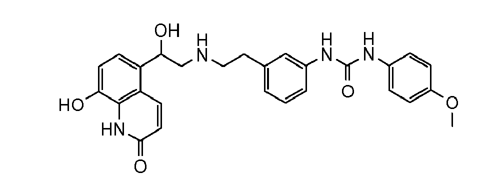

Obtained from Intermediate 66 (0.4, 0.69 mmol) and palladium on charcoal (0.1 g, 10%) by the same procedure described in Example 6 (reaction time: 24 hours). The crude was purified by column chromatography with silica gel, eluting with methylene chloride/methanol/ammonium (40:5:0.5) to obtain the title compound as a yellow solid (0.22 g, 64%). ¹H-NMR (300 MHz, dimethylsulfoxide-D6): 2.68-2.85 (m, 6H); 3.76 (s, 3H); 5.08 (bs, 1H); 6.56 (d, *J*=9.88 Hz, 1H); 6.83 (d, *J*=7.14 Hz, 1H); 6.90-6.97 (m, 3H); 7.11 (d, *J*=7.97 Hz, 1H); 7.18-7.31 (m, 2H); 7.36-7.42 (m, 3H); 8.22 (d, *J*=9.89 Hz, 1H); 8.52 (bs, 1H); 8.58 (bs, 1 H). MS(M+): 489.

### Intermediate 67. tert-butyl [2-(3-{[(biphenyl-4-ylamino)carbonyl]amino}phenyl)ethyl]-carbamate

Obtained from Intermediate 32 (0.708 g, 2.7 mmol) and biphenyl-4-amine (0.47 g, 2.97 mmol) by the same procedure described in Intermediate 33. The solvent was removed under reduced pressure to give the title compound, which was used in the next step without further manipulation.

### Intermediate 68. N-[3-(2-aminoethyl)phenyl]-N'-biphenyl-4-ylurea

Obtained from Intermediate 67 and hydrogen chloride saturated dioxane by the same procedure described in Intermediate 34. The crude was treated with acetonitrile, isopropylether and ether to give a precipitate, which was collected by filtration. The title compound was obtained as a solid (0.54 g, 60%) and used in the next step without further purification.

### Intermediate 69. N-{3-[2-({(2R,S)-2-[8-(benzyloxy)-2-oxo-1,2-dihydroquinolin-5-yl]-2-hydroxyethyl}amino)ethyl]phenyl}-N'-biphenyl-4-ylurea

Obtained from Intermediate 68 (0.54 g, 1.47 mmol), 8-(benzyloxy)-5-(2,2-dihydroxyacetyl)quinolin-2(1H)-one (0.4 g, 1.23 mmol), triethylamine (0.192 mL, 1.38 mmol) and sodium borohydride (0.12 g, 3.2 mmol) by the same procedure described in Intermediate 35. The crude was purified by column chromatography with silica gel, eluting with chloroform/methanol (from 10:0 to 8:1) giving the title compound as a solid (0.69 g, 77%).

### EXAMPLE 22. N-biphenyl-4-yl-N'-[3-(2-{[(2R,S)-2-hydroxy-2-(8-hydroxy-2-oxo-1,2-dihydroquinolin-5-yl)ethyl]amino}ethyl)phenyl]urea

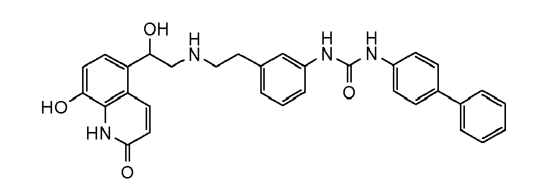

Obtained from Intermediate 69 (0.69 g, 1.10 mmol) and palladium on charcoal (0.17 g, 10%) by the same procedure described in Example 6 (reaction time: 48 hours). The crude was purified by column chromatography with silica gel, eluting with methylene chloride/methanol/ammonium (40:5:0.5). The title compound was obtained as a solid (0.344 g, 57%). ¹H-NMR (300 MHz, dimethylsulfoxide-D6): 2.67-2.81 (m, 6H); 4.05 (bs, 1H); 6.51 (d, *J*=9.9 Hz, 1H); 6.81 (d, *J*=7.15 Hz, 1H); 6.91 (d, *J*=7.97 Hz, 1H); 7.08 (d, *J*=8.24 Hz, 1H); 7.15-7.21 (m, 1H); 7.27-7.34 (m, 3H); 7.42-7.46 (m, 2H); 7.54-7.66 (m, 6H); 8.18 (d, *J*= 9.89 Hz, 1H); 8.69 (bs, 1H); 8.81 (bs, 1H). MS(M+): 535.

### Intermediate 70. tert-butyl {2-[3-({[(1-adamantylmethyl)amino]carbonyl}amino)-phenyl]ethyl}carbamate

Obtained from Intermediate 32 (0.661 g, 2.5 mmol) and 2-adamantan-1-yl-methylamine (0.44 g, 2.62 mmol) by the same procedure described in Intermediate 33. The solvent was removed under reduced pressure to give the title compound, which was used in the next step without further manipulation.

### Intermediate 71. N-(1-adamantylmethyl)-N'-[3-(2-aminoethyl)phenyl]urea

Obtained from Intermediate 70 and hydrogen chloride saturated dioxane by the same procedure described in Intermediate 34. The crude was treated with acetonitrile, isopropylether and ether to give a precipitate, which was collected by filtration. The title compound was obtained as a solid (0.426 g, 49%) and used in the next step without further purification.

### Intermediate 72. N-(1-adamantylmethyl)-N'-{3-[2-({(2R,S)-2-[8-(benzyloxy)-2-oxo-1,2-dihydroquinolin-5-yl]-2-hydroxyethyl}amino)ethyl]phenyl}urea

Obtained from Intermediate 71 (0.426 g, 1.1 mmol), 8-(benzyloxy)-5-(2,2-dihydroxyacetyl)quinolin-2(1H)-one (0.4 g, 1.23 mmol), sodium borohydride (0.1 g, 3.2 mmol) and triethylamine (0.154 mL, 1.1 mmol) by the same procedure described in Intermediate 35. The crude was purified by column chromatography with silica gel, eluting with chloroform/methanol (from 10:0 to 10:1). The title compound was obtained as a yellow foam (0.31 g, 44%).

### EXAMPLE 23. N-(1-adamantylmethyl)-N'-[3-(2-{[(2R,S)-2-hydroxy-2-(8-hydroxy-2-oxo-1,2-dihydroquinolin-5-yl)ethyl]amino}ethyl)phenyl]urea

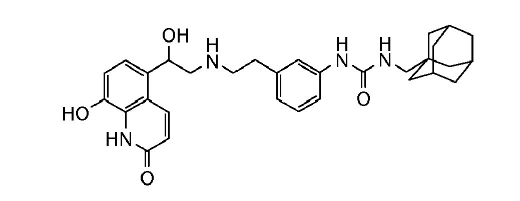

To a solution of Intermediate 72 (0.31 g, 0.5 mmol) in methanol was added hydrogen chloride 1.25 M in methanol until pH=5. Then 0,08 g of 10 % palladium on charcoal was added. The reaction mixture was hydrogenated under a balloon pressure at room temperature overnight. The catalyst was filtered through Celite® and the solvent removed under reduced pressure. The crude was treated with ether and the precipitate was collected by filtration and washed with ethyl acetate and acetonitrile to give the title compound as a solid (0.258 g, 87%). ¹H-NMR (300 MHz, dimethylsulfoxide-D6): 1.45 (bs, 5H); 1.57-1.69 (m, 5H); 1.93 (bs, 3H); 2.78 (bs, 2H); 2.89-3.20 (m, 6H); 5.40 (bs, 1H); 6.19 (bs, 1H); 6.32 (bs, 1H); 6.58 (d, *J*=9.7 Hz, 1H); 6.75 (bs, 1H); 6.99 (d, *J*=7.7 Hz, 1H); 7.15-7.18 (m, 3H); 7.39 (s, 1H); 8.22 (d, *J*=9.89 Hz, 1H); 8.67 (s, 1H); 8.78 (bs, 1H); 9.1 (bs, 1H); 10.47 (bs, 1H); 10.52 (bs, 1H). MS(M+): 568.

### Intermediate 73. tert-butyl {2-[3-({[(3-phenoxyphenyl)amino]carbonyl}amino)-phenyl]ethyl}carbamate

Obtained from Intermediate 32 (0.661 g, 2.5 mmol) and 3-phenoxyaniline (0.49 g, 2.62 mmol) by the same procedure described in Intermediate 33. The solvent was removed under reduced pressure to give the title compound, which was used in the next step without further manipulation.

### Intermediate 74. N-[3-(2-aminoethyl)phenyl]-N'-(3-phenoxyphenyl)urea

Obtained from Intermediate 73 and hydrogen chloride saturated dioxane by the same procedure described in Intermediate 34. The crude was treated with acetonitrile, isopropylether and ether to give a precipitate, which was collected by filtration. The title compound was obtained as a solid (0.45 g, 52%) and used in the next step without further purification.

### Intermediate 75. N-{3-[2-({(2R,S)-2-[8-(benzyloxy)-2-oxo-1,2-dihydroquinolin-5-yl]-2-hydroxyethyl}amino)ethyl]phenyl}-N'-(3-phenoxyphenyl)urea

Obtained from Intermediate 74 (0.45 g, 1.22 mmol), 8-(benzyloxy)-5-(2,2-dihydroxyacetyl)quinolin-2(1H)-one (0.4 g, 1.23 mmol), sodium borohydride (0.1 g, 3.2 mmol) and triethylamine (0.147 mL, 1.05 mmol) by the same procedure described in Intermediate 35. The crude was purified by column chromatography with silica gel, eluting with chloroform/methanol (from 10:0 to 10:1) to give the title compound as a yellow foam (0.59 g, 66%).

### EXAMPLE 24. N-[3-(2-{[(2R,S)-2-hydroxy-2-(8-hydroxy-2-oxo-1,2-dihydroquinolin-5-yl)ethyl]amino}ethyl)phenyl]-N'-(3-phenoxyphenyl)urea

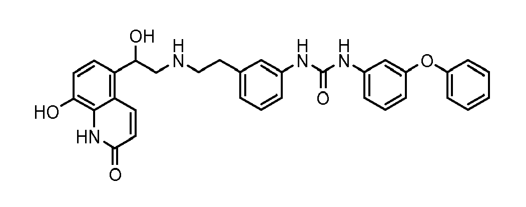

Obtained from Intermediate 75 (0.49 g, 0.72 mmol) and palladium on charcoal (0.1 g, 10%) by the same procedure described in Example 6 (reaction time: 24 hours). The crude was purified by column chromatography in reverse phase, eluting with acetonitrile/water to give the title compound as a solid (0.06 g, 15%). ¹H-NMR (300 MHz, dimethylsulfoxide-D6): 2.72-2.97 (m, 6H); 5.14 (bs, 1H); 6.52 (d, *J*=9.61 Hz, 1H); 6.59 (d, *J*=9.07 Hz, 1H); 6.8 (d, *J*=7.42 Hz, 1H); 6.93 (d, *J*=8.24 Hz, 1H); 7.02 (d, *J*=8.51 Hz, 1H); 7.08-7.2 (m, 5H); 7.23-7.31 (m, 3H); 7.37-7.43 (m, 3H); 8.16 (d, *J*=9.88 Hz, 1H); 9.26 (bs, 1H); 9.45 (bs, 1H). MS(M+): 551.

### Intermediate 76. 1-(3-(2-((2R)-2-(8-(benzyloxy)-2-oxo-1,2-dihydroquinolin-5-yl)-2-(tert-butyldimethylsilyloxy)ethylamino)ethyl)phenyl)-3-(3-phenylpropyl)urea

To a solution of Intermediate 40 (5.02 g, 16.88 mmol), *(R)*-8-(benzyloxy)-5-(2-bromo-1-(tert-butyldimethylsilyloxy)ethyl)quinolin-2(1 H)-one (5 g, 10.24 mmol) in N-methylpyrrolidinone (40 mL) was added sodium hydrogen carbonate (1.1 g, 12.5 mmol). The reaction mixture was stirred at 110°C for 8 hours. The crude reaction was poured into water and the precipitate was collected by filtration. The crude was purified by column chromatography with silica gel, eluting with methylene chloride/methanol (from 98:2 to 95:5) obtaining the title compound as a foam (3.3 g, 46%).

### Intermediate 77. N-{3-[2-({(2R)-2-[8-(benzyloxy)-2-oxo-1,2-dihydroquinolin-5-yl]-2-hydroxyethyl}amino)ethyl]phenyl}-N'-(3-phenylpropyl)urea

To a solution of Intermediate 76 (3.31 g, 4.7 mmol) in tetrahydrofuran (60 mL) was added tetrabutylammonium fluoride trihydrate (2.37 g, 7.51 mmol). The reaction mixture was stirred at 48°C for 4 hours. The solvent was removed under reduced pressure and the crude was partitioned between ethyl acetate and water. The organic layer was washed with water several times, dried and evaporated. The title compound was obtained as a solid (2.2 g, 79%).

### EXAMPLE 25. N-[3-(2-{[(2R)-2-hydroxy-2-(8-hydroxy-2-oxo-1,2-dihydroquinolin-5-yl)ethyl]amino}ethyl)phenyl]-N'-(3-phenylpropyl)urea

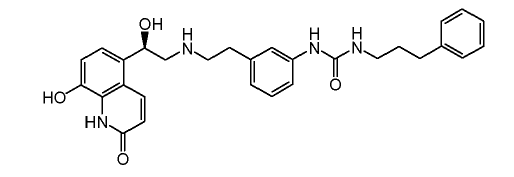

Obtained from Intermediate 77 (2.2 g, 3.72 mmol) and palladium on charcoal (0.4 g, 10%) by the same procedure described in Example 6 (reaction time: overnight). The crude was purified by column chromatography with silica gel, eluting with methylene chloride/methanol/ammonium (from 200:8:1 to 50:8:1) to give the title compound as a solid (0.64 g, 34%). ¹H-NMR (300 MHz, dimethylsulfoxide-D6): 1.67-1.77 (m, 2H); 2.57-2.80 (m, 7H); 3.05-3.1 (m, 3H); 5.00 (bs, 1H); 6.17 (bs, 1H); 6.49 (d, *J*=9.89 Hz, 1H); 6.71 (d, *J*=7.41 Hz, 1H); 6.90 (d, *J*=8.24 Hz, 1H); 7.04-7.12 (m, 2H); 7.17-7.31 (m, 7H); 8.16 (d, *J*=9.89 Hz, 1); 8.34 (bs, 1H). MS(M+): 501.

### Intermediate 78. diethyl (3-nitrobenzyl)malonate

To a solution of sodium (7 g, 0.3 mol) in ethanol (250 mL) was slowly added diethyl malonate (33 mL, 0.29 mol) and 1-(chloromethyl)-3-nitrobenzene (50 g, 0.29 mol). The reaction mixture was refluxed for 2 hours. The crude reaction was cooled and poured into a saturated solution of ammonium chloride. The organic phase was extracted with ether and washed with water and brine. The solvent was removed under reduced pressure obtaining the title compound as an oil (77.4 g, 91 %), which was used in the next step without further purification.

### Intermediate 79. ethyl 3-(3-nitrophenyl)propanoate

To a solution of Intermediate 78 (77.46 g, 0.26 mol) in dimethylsulfoxide (220 mL) was added lithium chloride (11 g, 0.26 mol) and water. The reaction mixture was stirred at 170°C for 5 hours. The crude reaction was poured into ice-water (1 L) and extracted with ethyl acetate. The organic layer was washed with water and brine, dried and the solvent was removed under reduced pressure giving the title compound as an oil (48.76 g, 84%), which was used in the next step without further purification.

### Intermediate 80. 3-(3-nitrophenyl)propanoic acid

To a solution of Intermediate 79 (48.76 g, 0.23 mol) in ethanol (50 mL) was added sodium hydroxide 2N (400 mL). The mixture was refluxed overnight. The crude reaction was cooled and the aqueous phase was treated with hydrochloric acid and extracted with ether. The solvent was removed under reduced pressure obtaining an oil, which was purified by column chromatography with silica gel, eluting with methylene chloride/methanol (98:2). The title compound was obtained as a solid (8.13 g, 18%).

### Intermediate 81. [2-(3-nitrophenyl)ethyl]amine

To a solution of Intermediate 80 (7.75 g, 39.71 mmol) in sulphuric acid (19 mL) was added chloroform (10 mL). The mixture was stirred at 50°C and sodium azide (3.8 g, 58.45 mmol) was slowly added (addition time: 2 hours). The reaction was stirred at 50°C for 2 hours. The crude was poured into potassium carbonate (40 g) to neutralize the reaction, then water was added (30 mL) and the crude was extracted with ethyl acetate. The crude was purified by column chromatography with silica gel, eluting with methylene chloride/methanol (from 98:2 to 9:1) to give the title compound as an oil (3.92 g, 59%).

### Intermediate 82. 8-(benzyloxy)-5-((1R)-1-{[tert-butyl(dimethyl)silyl]oxy}-2-{[2-(3-nitrophenyl)ethyl]amino}ethyl)quinolin-2(H)-one

To a solution of Intermediate 81 (3.27 g, 19.68 mmol) and *(R)*-8-(benzyloxy)-5-(2-bromo-1-(tert-butyldimethylsilyloxy)ethyl)quinolin-2(1H)-one (5 g, 10.24 mmol) in N-methylpyrrolidinone (625 mL) was added sodium iodide (1.5 g, 10.21 mmol) and sodium hydrogen carbonate (1.7 g, 19.64 mmol). The reaction mixture was heated in a sealed tub at 110°C for 4 hours. The crude reaction was poured into water and extracted with ethyl acetate. The organic layer was washed with water and brine. The solvent was removed under reduced pressure and the crude obtained was purified by column chromatography with silica gel, eluting with methylene chloride/methanol (98:2) to give the title compound as an oil (3.76 g, 64%).

### Intermediate 83. tert-butyl ((2R)-2-[8-(benzyloxy)-2-oxo-1,2-dihydroquinolin-5-yl]-2-{[tert-butyl(dimethyl)silyl]oxy}ethyl)[2-(3-nitrophenyl)ethyl]carbamate

To a solution of Intermediate 82 (3.76 g, 6.55 mmol) in methylene chloride (60 mL) was added di-tert-butyl dicarbonate (1.43 g, 6.55 mmol) and triethylamine (0.9 mL, 6.53 mmol). The reaction mixture was stirred at room temperature for 2 hours. The solvent was removed under reduced pressure and the crude was purified by column chromatography with silica gel, eluting with methylene chloride/ethanol (from 100:0 to 99:1). The title compound was obtained as an oil (4.52 g, 72%).

### Intermediate 84. tert-butyl [2-(3-aminophenyl)ethyl]((2R)-2-[8-(benzyloxy)-2-oxo-1,2-dihydroquinolin-5-yl]-2-{[tert-butyl(dimethyl)silyl]oxy}ethyl)carbamate

To a solution of Intermediate 83 (3.52 g, 5.22 mmol) in methanol (80 mL) was added Ni-Raney. The reaction mixture was hydrogenated under a balloon pressure for 1 hour. The catalyst was filtered through Celite® and the solvent was removed under reduced pressure. The title compound was obtained as a foam (3 g, 89%) and used in the next step without further purification.

### Intermediate 85. tert-butyl ((2R)-2-[8-(benzyloxy)-2-oxo-1,2-dihydroquinolin-5-yl]-2-{[tert-butyl(dimethyl)silyl]oxy}ethyl){2-[3-({[(diphenylmethyl)amino]carbonyl}amino)-phenyl]ethyl}carbamate

To a solution of Intermediate 84 (4.16 g, 5.62 mmol) in tetrahydrofuran (130 mL) was added under nitrogen benzhydryl isocyanate (1.45 mL, 7.6 mmol). The reaction mixture was stirred at room temperature for 24 hours. The crude was purified by column chromatography with silica gel, eluting with chloroform/methanol (from 100:0 to 95:1) to give the title compound as a foam (3.83 g, 73%).

### Intermediate 86. N-{3-[2-({(2R)-2-[8-(benzyloxy)-2-oxo-1,2-dihydroquinolin-5-yl]-2-hydroxyethyl}amino)ethyl]phenyl}-N'-(diphenylmethyl)urea

A solution of Intermediate 85 (3.6 g, 4.25 mmol) in hydrogen chloride 4M in dioxane (90 mL) was stirred at room temperature for 2.5 hours. The crude obtained was purified by column chromatography with silica gel, eluting with chloroform/methanol (from 50:1 to 15:1) to give the title compound as a yellow foam (2.3 g, 83%).

### EXAMPLE 26. N-(diphenylmethyl)-N'-[3-(2-{[(2R)-2-hydroxy-2-(8-hydroxy-2-oxo-1,2-dihydroquinolin-5-yl)ethyl]amino}ethyl)phenyl]urea

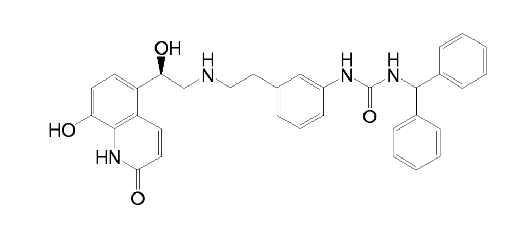

Obtained from Intermediate 86 (2.3 g, 3.60 mmol) and palladium on charcoal (0.19 g, 10%) by the same procedure described in Example 6 (reaction time: 2 days). The crude obtained was treated with ether and the precipitate was collected by filtration and washed with ethyl acetate to give the title compound as a solid (1.3 g, 64%). ¹H-NMR (300 MHz, dimethylsulfoxide-D6): 2.89-3.22 (m, 6H); 5.40 (bs, 1 H); 5.95 (bs, 1H); 6.18 (bs, 1H); 6.58 (d, *J*=9.89 Hz, 1H); 6.78 (bs, 1H); 7.00 (d, *J*=7.96 Hz, 1H); 7.16-7.25 (m, 3H); 7.32-7.4 (m, 10H); 7.51 (d, *J*=8.51 Hz, 1H); 8.21 (d, *J*=9.89 Hz, 1H); 8.77 (bs, 1H); 8.9 (bs, 1H); 9.08 (bs, 1H); 10.5 (bs, 1H). MS(M+): 549.

### Intermediate 87. tert-butyl ((2R)-2-[8-(benzyloxy)-2-oxo-1,2-dihydroquinolin-5-yl]-2-{[tert-butyl(dimethyl)silyl]oxy}ethyl)[2-(3-{[(biphenyl-2-ylamino)carbonyl]amino}phenyl)ethyl]carbamate

Obtained from Intermediate 84 (1.8 g, 2.74 mmol) and 2-isocyanatobiphenyl (0.52 mL, 3.02 mmol) by the same procedure described in Intermediate 85. The crude was purified by column chromatography with silica gel, eluting with methylene chloride/methanol (from 100:1 to 99:1) to give the title compound as a solid (2 g, 85%).

### Intermediate 88. N-{3-[2-({(2R)-2-[8-(benzyloxy)-2-oxo-1,2-dihydroquinolin-5-yl]-2-hydroxyethyl}amino)ethyl]phenyl}-N'-biphenyl-2-ylurea

Obtained from Intermediate 87 (2g, 2.34 mmol) and hydrogen chloride 4M in dioxane (35 mL) by the same procedure described in Intermediate 86. The crude was purified by column chromatography with silica gel, eluting with methylene chloride/methanol (from 98:2 to 95:5) to give the tile compound as a solid (1 g, 71%).

### EXAMPLE 27. N-biphenyl-2-yl-N'-[3-(2-{[(2R)-2-hydroxy-2-(8-hydroxy-2-oxo-1,2-dihydroquinolin-5-yl)ethyl]amino}ethyl)phenyl]urea

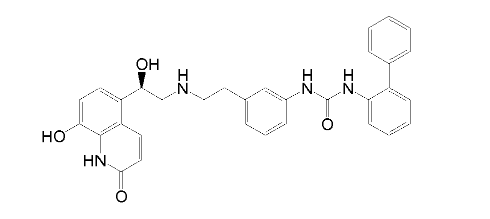

Obtained from Intermediate 88 (1.44 g, 2.30 mmol) and palladium on charcoal (0.25 g, 10%) by the same procedure described in Example 6 (reaction time: 2 hours). The crude obtained was purified by column chromatography in reverse phase, eluting with acetonitrile/water. The title compound was obtained as solid (0.9 g, 73%). ¹H-NMR (300 MHz, dimethylsulfoxide-D6): 2.69-2.88 (m, 6H); 5.12 (bs, 1 H); 6.51 (d, *J*=9.89 Hz, 1); 6.78 (d, *J*=7.42 Hz, 1 H); 6.96 (d, *J*=7.96 Hz, 1 H); 7.07-7.35 (m, 7H); 7.4-7.51 (m, 5H); 7.89 (bs, 1 H); 8.19 (d, *J*=9.89 Hz, 1 H); 9.23 (bs, 1 H). MS(M+): 535.

### Intermediate 89. N-(3-{2-[((2R)-2-[4-(benzyloxy)-3-(formylamino)phenyl]-2-{[tert-butyl(dimethyl)silyl]oxy}ethyl)amino]ethyl}phenyl)-N'-biphenyl-2-ylurea

To a solution of Intermediate 56 (0.79 g, 2.38 mmol) and (R)-N-(2-(benzyloxy)-5-(2-bromo-1-(tert-butyldimethylsilyloxy)ethyl)phenyl)formamide (1.12 g, 2.41 mmol) in dimethylsulfoxide (3 mL) was added sodium hydrogen carbonate (0.6 g, 7.14 mmol) and sodium iodide (0.4 g, 2.74 mmol). The reaction mixture was stirred in a sealed tub at 110°C for 1 hour. The reaction crude was poured into water and the precipitate was collected by filtration. The crude obtained was purified by column chromatography with silica gel, eluting with methylene chloride/methanol (from 100:0 to 95:5) to give the title compound as a solid (0.65 g, 38%).

### Intermediate 90. N-{3-[2-({(2R)-2-[4-(benzyloxy)-3-(formylamino)phenyl]-2-hydroxyethyl}amino)ethyl]phenyl}-N'-biphenyl-2-ylurea

Obtained from Intermediate 89 (0.65 g, 0.91 mmol) and tetrabutylammonium trihydrate (0.43 g, 1.64 mmol) by the same procedure described in Intermediate 77. The crude obtained was purified by column chromatography with silica gel, eluting with methylene chloride/methanol (form 100:0 to 95:5) to give the title compound as a solid (0.25 g, 45%).

### EXAMPLE 28. N-biphenyl-2-yl-N'-{3-[2-({(2R)-2-[3-(formylamino)-4-hydroxyphenyl]-2-hydroxyethyl}amino)ethyl]phenyl}urea

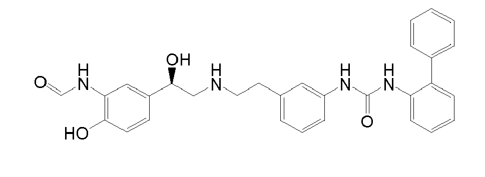

To a solution of Intermediate 90 (0.25 g, 0.42 mmol) in methanol (10 mL) was added palladium on charcoal (0.04 g, 10%). The reaction mixture was hydrogenated at 30 psi at room temperature overnight. The catalyst was filtered through Celite® and the solvent removed under reduced pressure. The crude was treated with ether and the title compound was obtained as a solid (0.136 g, 71%). ¹H-NMR (300 MHz, dimethylsulfoxide-D6): 2.59-2.82 (m, 6H); 4.5 (bs, 1H); 6.78-6.87 (m, 3H); 7.06-7.22 (m, 5H); 7.3-7.52 (m, 6H); 7.64 (bs, 1H); 7.92 (d, *J*=7.69 Hz, 1H); 8.02 (bs, 1H); 8.25 (bs, 1H); 8.97 (bs, 1H); 9.54 (bs, 1H). MS(M+): 511.

### Intermediate 91. diethyl methyl(3-nitrobenzyl)malonate

Obtained from 1-(chloromethyl)-3-nitrobenzene (50 g, 0.29 mol), diethyl 2-methylmalonate (53 g, 0.3 mol) and sodium (7 g, 0.3 mol) by the same procedure described in Intermediate 78. The crude was obtained as an oil (87.99 g, 80%) and used in the next step without further purification.

### Intermediate 92. ethyl (2R,S)-2-methyl-3-(3-nitrophenyl)propanoate

Obtained from Intermediate 91 (87.96 g, 0.23 mol) and lithium chloride (22.98 g, 0.54 mol) by the same procedure described in Intermediate 79 (reaction time: 5.5 hours). The title compound was obtained as a brown oil (55.25 g, 85%) and used in the next step without further purification.

### Intermediate 93. (2R,S)-2-methyl-3-(3-nitrophenyl)propanoic acid

Obtained from Intermediate 92 (55.24 g, 0.2 mol) and sodium hydroxide 2N (468 mL, 0.94 mol) by the same procedure described in Intermediate 80. The crude obtained was treated with hexane and the precipitate was collected by filtration and washed with hexane to give the title compound as an orange-brown solid (42.31 g, 97%), which was used in the next step without further purification.

### Intermediate 94. [(1R,S)-1-methyl-2-(3-nitrophenyl)ethyl]amine

Obtained from Intermediate 93 (30.3 g, 0.14 mol), sodium azide (13.4 g, 0.21 mol) and sulphuric acid (130 mL, 95-98%) by the same procedure described in Intermediate 81. The title compound was obtained as a brown oil (24.7 g, 91%).

### Intermediate 95. [1-methyl-2-(3-nitrophenyl)ethyl]amine, D-enantiomer

To a solution of Intermediate 94 (racemic amine) (27 g, 0.15 mol) in methanol (1140 mL) was added (L)-tartaric acid (22.51 g, 0.15 mol). The mixture was refluxed for 5 minutes and stirred at room temperature for 4.5 hours. The precipitate 1 was collected by filtration and washed with cool methanol (100 mL) and ether (x2). The crude obtained was a white-yellow solid (33.9 g), which was recristalized in methanol (1150 mL). A precipitate **2** was obtained, collected by filtration and washed with cool methanol (50 mL) and ether (x2). The precipitate **2** was dissolved in water and potassium carbonate was added, after 30 minutes stirring the crude was extracted twice with chloroform, obtaining the title compound as the dextrorotatory amine (15.13 g; [α]=+19.2°; c=0.01 g/mL).

### Intermediate 96. [1-methyl-2-(3-nitrophenyl)ethyl]amine, L-enantiomer

The solvent of the filtrate from the precipitates **1** and **2** (Intermediate 95) was removed under reduced pressure giving a white-yellow solid, which was treated with solid potassium carbonate in water and extracted twice with chloroform giving the free base amine. To a solution of amine (14.67 g, 0.08 mol) in methanol (550 mL) was added (D)-tartaric acid (12.22 g, 0.08 mol). The mixture was refluxed for 5 minutes and stirred at room temperature overnight. The precipitate was collected by filtration and washed with a mixture of cool methanol/ether 1:1 (50 mL) giving a pale yellow solid (18.1 g). The solid was dissolved in water and potassium carbonate was added, after 30 minutes stirring the crude was extracted twice with chloroform, giving the title compound as the levorotatory amine (14.7 g, [α]=-18.3°; c=0.01 g/mL).

### Intermediate 97. 8-(benzyloxy)-5-((1R)-1-{[tert-butyl(dimethyl)silyl]oxy}-2-{[1-methyl-2-(3-nitrophenyl)ethyl]amino}ethyl)quinolin-2(1H)-one, a isomer

Obtained from Intermediate 96 (1.37 g, 7.6 mmol), (*R*)-8-(benzyloxy)-5-(2-bromo-1-(tert-butyldimethylsilyloxy)ethyl)quinolin-2(1 H)-one (3.1 g, 6.35 mmol), sodium hydrogen carbonate (0.6 g, 7.62 mmol) and sodium iodide (1 g, 6.34 mmol) by the same procedure described in Intermediate 82 (reaction time: 6 hours). The crude obtained was purified by column chromatography with silica gel, eluting with methylene chloride/methanol (from 100:0 to 97:1) to give the title compound as a solid (2.21 g, 59%).

### Intermediate 98. tert-butyl ((2R)-2-[8-(benzyloxy)-2-oxo-1,2-dihydroquinolin-5-yl]-2-{[tert-butyl(dimethyl)silyl]oxy}ethyl)[1-methyl-2-(3-nitrophenyl)ethyl]carbamate, a isomer

Obtained from Intermediate 97 (2.2 g, 3.74 mmol), di-tert-butyl dicarbonate (0.82 g, 3.76 mmol) and triethylamine (0.5 mL, 3.73 mmol) by the same procedure described in Intermediate 83. The crude was purified by column chromatography with silica gel, eluting with methylene chloride/methanol (from 100:0 to 99:1) to give the title compound as a foam (2 g, 78%).

### Intermediate 99. tert-butyl [2-(3-aminophenyl)-1-methylethyl]((2R)-2-[8-(benzyloxy)-2-oxo-1,2-dihydroquinolin-5-yl]-2-{[tert-butyl(dimethyl)silyl]oxy}ethyl)carbamate, a isomer

Obtained from Intermediate 98 (2.2 g, 3.2 mmol) and Ni-Raney (0.2 g, 3.41 mmol) by the same procedure described in Intermediate 84. The solvent was removed under reduced pressure giving the title compound as a foam (1.8 g, 85%), which was used in the next step without further purification.

### Intermediate 100. tert-butyl ((2R)-2-[8-(benzyloxy)-2-oxo-1,2-dihydroquinolin-5-yl]-2-{[tert-butyl(dimethyl)silyl]oxy}ethyl)[2-(3-{[(biphenyl-2-ylamino)carbonyl]amino}-phenyl)-1-methylethyl]carbamate, a isomer

Obtained from Intermediate 99 (1.8 g, 2.74 mmol) and 2-isocyanatobiphenyl (0.52 mL, 3.02 mmol) by the same procedure described in Intermediate 85. The crude was purified by column chromatography with silica gel, eluting with methylene chloride/methanol (from 100:0 to 99:1) giving the title compound as a solid (2 g, 85%).

### Intermediate 101. N-{3-[2-({(2R)-2-[8-(benzyloxy)-2-oxo-1,2-dihydroquinolin-5-yl]-2-hydroxyethyl}amino)propyl]phenyl}-N'-biphenyl-2-ylurea, a isomer

Obtained from Intermediate 100 (2 g, 2.34 mmol) and hydrogen chloride 4M in dioxane (35 mL) by the same procedure described in Intermediate 86 (reaction time: 3 hours). The crude obtained was purified by column chromatography with silica gel, eluting with methylene chloride/methanol (form 98:2 to 95:5) to give the title compound as a solid (1 g, 71 %).

### EXAMPLE 29. N-biphenyl-2-yl-N'-[3-(2-{[(2R)-2-hydroxy-2-(8-hydroxy-2-oxo-1,2-dihydroquinolin-5-yl)ethyl]amino}propyl)phenyl]urea, a isomer

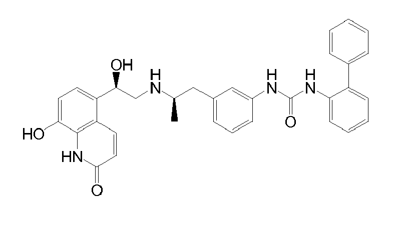

Obtained from Intermediate 101 (1 g, 1.57 mmol) and palladium on charcoal (0.17 g, 10%) by the same procedure described in Example 6 (reaction time: 2 hours). The crude was treated with ether and the precipitate was collected by filtration giving the title compound as a solid (0.8 g, 92%). ¹H-NMR (300 MHz, dimethylsulfoxide-D6): 1.1 (d, *J*=5.77 Hz, 3H); 2.6-2.67 (m, 1 H); 3.06-3.28 (m, 4H); 5.52 (bs, 1H); 6.59 (d, *J*=9.89 Hz, 1H); 6.83 (bs, 1H); 7.01 (d, *J*=8.24 Hz, 1H); 7.12-7.22 (m, 5H); 7.3-7.52 (m, 7H); 7.74 (s, 1H); 7.91 (d, *J*=7.96 Hz, 1H); 8.31 (d, *J*=9.89 Hz, 1H); 8.73 (bs, 1H); 9.14 (s, 1H); 9.49 (bs, 1H); 10.5 (bs, 1 H). MS(M+): 549.

### Intermediate 102. tert-butyl [1-methyl-2-(3-nitrophenyl)ethyl]carbamate, b isomer

To a solution of Intermediate 95 (2.1 g, 11.65 mmol) in tetrahydrofuran (30 mL) was added dropwise di-tert-butyl dicarbonate (2.54 g, 11.64) in tetrahydrofuran (15 mL). The reaction mixture was stirred at room temperature overnight. The solvent was removed under reduced pressure and the crude was partitioned between ciclohexane/water. The organic layer was washed with water (x3), dried and evaporated. The title compound was obtained as a white solid (3.3 g, 99%) and used in the next step without further purification.

### Intermediate 103. tert-butyl [2-(3-aminophenyl)-1-methylethyl]carbamate, b isomer

To a solution of Intermediate 102 (3.3 g, 11.77 mmol) in methanol (125 mL) was added palladium on charcoal (0.31 g, 10%). The reaction mixture was hydrogenated under a balloon pressure for 4 hours at room temperature. The catalyst was filtered through Celite® and the solvent removed under reduced pressure. The title compound was obtained as an oil (2.43 g, 79%) and used in the next step without further purification.

### Intermediate 104. tert-butyl [2-(3-{[(biphenyl-2-ylamino)carbonyl]amino}phenyl)-1-methylethyl]carbamate, b isomer

Obtained from Intermediate 103 (2 g, 8.19 mmol) and 2-isocyanatobiphenyl (1.4 mL, 8.18 mmol) by the same procedure described in Intermediate 4. The crude was treated with hexane and ether obtaining a precipitate, which was collected by filtration to give the title compound as a grey-white solid (3.4 g, 93%).

### Intermediate 105. N-{3-[2-aminopropyl]phenyl}-N'-biphenyl-2-ylurea, b isomer

To a solution of Intermediate 104 (3.4 g, 7.73 mmol) in tetrahydrofuran (31.6 mL) was added hydrochloric acid (31.6 mL, 35%). The mixture was stirred at room temperature for 75 minutes. The solvent was removed under reduced pressure and the aqueous residue was treated with potassium carbonate and extracted with chloroform (x2). The crude (3 g) obtained was dissolved in hydrogen chloride 1.25 M in ethanol (14 mL) and stirred for some minutes. The solvent was removed under reduced pressure and the title compound was obtained as a salt (2.7 g, 91%).

### Intermediate 106. N-biphenyl-2-yl-N'-[3-(2-{[(2R)-2-{[tert-butyl(dimethyl)silyl]oxy}-2-(2,2-dimethyl-4H-1,3-benzodioxin-6-yl)ethyl]amino}propyl)phenyl]urea, b isomer

To a solution of Intermediate 105 (0.9 g, 2.61 mmol), (*R*)-(2-bromo-1-(2,2-dimethyl-4H-benzo[d][1,3]dioxin-6-yl)ethoxy)(tert-butyl)dimethylsilane (0.8 g, 2.09 mmol) in *N-*methylpyrrolidinone (4.13 mL) was added sodium hydrogen carbonate (0.22 g, 2.6 mmol). The reaction mixture was stirred in a sealed tub at 120°C for 2.5 hours. The reaction crude was poured into water and extracted with ethyl acetate. The solvent was removed under reduced pressure and the crude obtained was purified by column chromatography with silica gel, eluting with chloroform/methanol (from 75:1 to 25:1). The title compound was obtained as an oil (0.63 g, 39%).

### Intermediate 107. N-biphenyl-2-yl-N'-[3-(2-{[(2R)-2-(2,2-dimethyl-4H-1,3-benzodioxin-6-yl)-2-hydroxyethyl]amino}propyl)phenyl]urea, b isomer

Obtained from Intermediate 106 (0.87 g, 1.14 mmol) and tetrabutylammonium trihydrate (0.7 g, 2.23 mmol) by the same procedure described in Intermediate 77. The crude was purified by column chromatography with silica gel, eluting with chloroform/methanol (from 25:1 to 15:1) to give the title compound as a yellow foam (0.59 g, 93%).

### EXAMPLE 30. N-biphenyl-2-yl-N'-{3-[2-({(2R)-2-hydroxy-2-[4-hydroxy-3-(hydroxymethyl)phenyl]ethyl}amino)propyl]phenyl}urea, b isomer

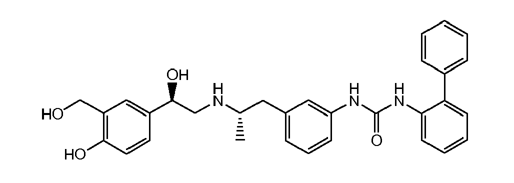

A solution of Intermediate 107 (0.48 g, 0.88 mmol) in a mixture of acetic acid/water (4.45 mL/2.23 mL) was stirred at 80°C for 30 minutes. The solvent was removed under reduced pressure and the crude was purified by column chromatography with silica gel, eluting with chloroform/methanol/ammonium (from 90:10/0.5 to 90/10/1). The title compound was obtained as a white solid (0.37 g, 83%). ¹H-NMR (300 MHz, dimethylsulfoxide-D6): 0.91 (d, *J*=5.76 Hz, 3H); 2.38-2.45 (m, 1H); 2.61-2.68 (m, 3H); 2.76-2.82 (m, 1H); 4.93 (bs, 1H); 5.01 (bs, 1H); 6.67 (d, *J*=8.24 Hz, 1H); 6.76 (d, *J*= 6.59 Hz, 1H); 6.96 (d, *J*=8.24 Hz, 1H); 7.1-7.25 (m, 6H); 7.33 (t, *J*=6.32 Hz, 1H); 7.39-7.44 (m, 3H); 7.49-7.54 (m, 2H); 7.63 (s, 1H); 7.94 (d, *J*=8.24 Hz, 1H); 8.98 (bs, 1H); 9.17 (bs, 1H). MS(M+): 512.

### Intermediate 108. tert-butyl [1-methyl-2-(3-nitrophenyl)ethyl]carbamate, a isomer

Obtained from Intermediate 96 (2.14 g, 11.88 mmol) and di-tert-butyl dicarbonate (2.59 g, 11.87 mmol) by the same procedure described in Intermediate 102. The title compound was obtained as a pale yellow solid (3.1 g, 93%) and used in the next step without further purification.

### Intermediate 109. tert-butyl [2-(3-aminophenyl)-1-methylethyl]carbamate, a isomer

Obtained from Intermediate 108 (3.1 g, 11.12 mmol) and palladium on charcoal (0.31 g, 10%) by the same procedure described in Intermediate 103. The title compound was obtained as a solid (2.78 g, 96%) and used in the next step without further purification.

### Intermediate 110. tert-butyl [2-(3-{[(biphenyl-2-ylamino)carbonyl]amino}phenyl)-1-methylethyl]carbamate, a isomer

Obtained from Intermediate 109 (2.7 g, 11.1 mmol) and 2-isocyanatobiphenyl (1.9 mL, 11.1 mmol) by the same procedure described in Intermediate 4 (reaction time:
overnight). The title compound was obtained as a foam (4.1 g, 84%) and used in the next step without further purification.

### Intermediate 111. N-{3-[2-aminopropyl]phenyl}-N'-biphenyl-2-ylurea, a isomer

Obtained from Intermediate 110 (4.1 g, 9.4 mmol) and hydrochloric acid (38 mL, 35%) by the same procedure described in Intermediate 105. The title compound was obtained as a solid (2.9 g, 89%) and used in the next step without further purification.

### Intermediate 112. N-biphenyl-2-yl-N'-[3-(2-{[(2R)-2-{[tert-butyl(dimethyl)silyl]oxy}-2-(2,2-dimethyl-4H-1,3-benzodioxin-6-yl)ethyl]amino}propyl)phenyl]urea, a isomer

Obtained from Intermediate 111 (1.2 g, 3.6 mmol), (*R*)-(2-bromo-1-(2,2-dimethyl-4H-benzo[d][1,3]dioxin-6-yl)ethoxy)(tert-butyl)dimethylsilane (1.1 g, 2.91 mmol) and sodium hydrogen carbonate (0.3 g, 3.63 mmol) by the same procedure described in Intermediate 106. The crude was purified by column chromatography with silica gel, eluting with methylene chloride/methanol (from 75:1 to 25:1) to give the title compound as solid (0.9 g, 44%).

### Intermediate 113. N-biphenyl-2-yl-N'-[3-(2-{[(2R)-2-(2,2-dimethyl-4H-1,3-benzodioxin-6-yl)-2-hydroxyethyl]amino}propyl)phenyl]urea, a isomer

Obtained from Intermediate 112 (0.9 g, 1.35 mmol) and tetrabutylammonium trihydrate (0.85 g, 2.71 mmol) by the same procedure described in Intermediate 77. The crude was purified by column chromatography with silica gel, eluting with chloroform/methanol (15:1) to give the title compound as a white foam (0.59 g, 79%).

### EXAMPLE 31. N-biphenyl-2-yl-N'-{3-[2-({(2R)-2-hydroxy-2-[4-hydroxy-3-(hydroxymethyl)phenyl]ethyl}amino)propyl]phenyl}urea, a isomer

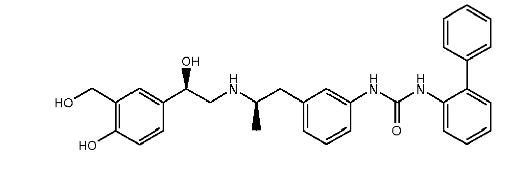

Obtained from Intermediate 113 (0.59 g, 1.07 mmol) in a mixture of acetic acid/water (5.4 mL/2.7 mL) by the same procedure described in Example 30. The crude was purified by column chromatography with silica gel, eluting with chloroform/methanol/ammonium (from 90/10/0.5 to 80/20/2) giving the title compound as a white solid (0.49 g, 90%). ¹H-NMR (300 MHz, dimethylsulfoxide-D6): 0.91 (d, *J*=6.04 Hz, 3H); 2.31-2.39 (m, 1H); 2.61-2.83 (m, 4H); 4.46 (s, 2H); 4.97 (bs, 1H); 6.68 (d, *J*=8.24 Hz, 1H); 6.74 (d, *J*= 7.42 Hz, 1H); 6.99 (d, *J*=8.24 Hz, 1H); 7.11-7.15 (m, 2H); 7.2-7.25 (m, 4H); 7.33 (t, *J*=8.24 Hz, 1H); 7.40-7.45 (m, 3H); 7.49-7.54 (m, 1H); 7.63 (s, 1H); 7.94 (d, *J*=8.24 Hz, 1H); 8.97 (s, 1H); 9.17 (bs, 1H). MS(M+): 512.

### Intermediate 114. N-(3-{2-[((2R)-2-[8-(benzyloxy)-2-oxo-1,2-dihydroquinolin-5-yl]-2-{[tert-butyl(dimethyl)silyl]oxy}ethyl)amino]propyl}phenyl)-N'-biphenyl-2-ylurea, b isomer

Obtained from Intermediate 105 (0.71 g, 2.06 mmol), *(R)*-8-(benzyloxy)-5-(2-bromo-1-(tert-butyldimethylsilyloxy)ethyl)quinolin-2(1 H)-one (1 g, 2.05 mmol), sodium hydrogen carbonate (0.2 g, 2.62 mmol) and sodium iodide (0.3 g, 2.07 mmol) by the same procedure described in Intermediate 82 (reaction time: 7 hours). The crude was purified by column chromatography with silica gel, eluting with methylene chloride/methanol (from 100:0 to 95:5) to give the title compound as a foam (0.58 g, 36%).

### Intermediate 115. N-{3-[2-({(2R)-2-[8-(benzyloxy)-2-oxo-1,2-dihydroquinolin-5-yl]-2-hydroxyethyl}amino)propyl]phenyl}-N'-biphenyl-2-ylurea, b isomer

Obtained from Intermediate 114 (0.58 g, 0.77 mmol) and tetrabutylammonium trihydrate (0.4 g, 1.53 mmol) by the same procedure described in Intermediate 77. The crude was purified by column chromatography with silica gel, eluting with methylene chloride/methanol (from 98:2 to 95:5) to give the title compound as a solid (0.38 g, 77%).

### EXAMPLE 32. N-biphenyl-2-yl-N'-[3-(2-{[(2R)-2-hydroxy-2-(8-hydroxy-2-oxo-1,2-dihydroquinolin-5-yl)ethyl]amino}propyl)phenyl]urea, b isomer

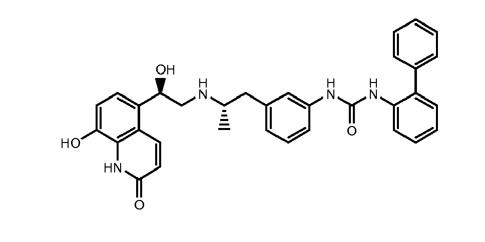

Obtained from Intermediate 115 (0.38 g, 0.59 mmol) and palladium on charcoal (0.15 g, 10%) by the same procedure described in Example 6. The crude obtained was purified by column chromatography with silica gel, eluting with methylene chloride/methanol (from 98:2 to 95:5) to give the title compound as a solid (0.16 g, 51%). ¹H-NMR (300 MHz, dimethylsulfoxide-D6): 0.92 (d, *J*=6.04 Hz, 3H); 2.39-2.47 (m, 1H); 2.65-2.89 (m, 4H); 4.99 (bs, 1H); 6.51 (d, *J*=9.89 Hz, 1H); 6.75 (d, *J*=6.86 Hz, 1H); 6.9 (d, *J*=8.24 Hz, 1H); 7.005 (d, *J*=8.24 Hz, 1H); 7.1-7.25 (m, 5H); 7.33 (t, *J*=7.14 Hz, 1H); 7.4-7.53 (m, 4H); 7.64 (s, 1H); 7.94 (d, *J*=8.24 Hz, 1H); 8.16 (d, *J*=9.82 Hz, 1H); 8.99 (s, 1H). MS(M+): 549.

### Intermediate 116. tert-butyl {2-[3-({[(4-tert-butylbenzyl)amino]carbonyl}amino)-phenyl]ethyl}carbamate

Obtained from Intermediate 32 (0.6 g, 2.5 mmol) and (3-*tert*-butylphenyl)methanamine (0.43 g, 2.6 mmol) by the same procedure described in Intermediate 33. The solvent was evaporated and the crude was used in the next step without further purification.

### Intermediate 117. N-[3-(2-aminoethyl)phenyl]-N'-(4-tert-butylbenzyl)urea

Obtained from Intermediate 116 by the same procedure described in Intermediate 34. The crude was purified by column chromatography with silica gel, eluting with methylene chloride/methanol/ammonium (from 40:2.5:0.1 to 40:8:1) to give the title compound (0.35 g, 32%).

### Intermediate 118. N-{3-[2-({2-[8-(benzyloxy)-2-oxo-1,2-dihydroquinolin-5-yl]-2-hydroxyethyl}amino)ethyl]phenyl}-N'-(4-tert-butylbenzyl)urea

Obtained from Intermediate 117 (0.35 g, 1.07 mmol), 8-(benzyloxy)-5-(2,2-dihydroxyacetyl)quinolin-2(1 H)-one (0.33 g, 1.01 mmol), triethylamine (0.141 mL, 1.12 mmol) and sodium borohydride (0.1 g, 1.17 mmol) by the same procedure described in Intermediate 35. The title compound was used without further purification (0.19 g, 40%).

### Example 33. N-(4-tert-butylbenzyl)-N'-[3-(2-{[(2R,S)-2-hydroxy-2-(8-hydroxy-2-oxo-1,2-dihydroquinolin-5-yl)ethyl]amino}ethyl)phenyl]urea

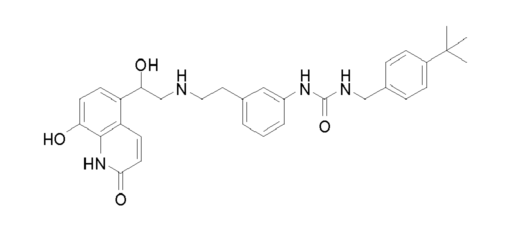

Obtained from Intermediate 118 (0.19 g, 0.31 mmol) and palladium on charcoal (0.04 g, 10%) by the same procedure described in Example 6 (reaction time: 6 hours). The curde obtained was treated with ethyl acetate and the solid was separated by filtration giving the title compound (0.142 g, 82%). ¹H-NMR (300 MHz, dimethylsulfoxide-D6): 1.26 (bs, 9H); 2.89-3.19 (m, 6H); 4.24 (s, 2H); 5.42 (bs, 1H); 6.56 (d, *J*=9.89 Hz, 1H); 6.78 (bs, 2H); 7.01 (d, *J*=8.24 Hz, 1H); 7.14-7.23 (m, 6H); 7.33-7.4 (m, 3H); 8.23 (d, *J*=9.82 Hz, 1 H); 8.8 (s, 1H); 9.19 (bs, 1H); 10.51 (bs, 1H). MS(M+): 529.

### Intermediate 119. 8-(benzyloxy)-5-((1R)-1-{[tert-butyl(dimethyl)silyl]oxy}-2-{[2-(4-nitrophenyl)ethyl]amino}ethyl)quinolin-2(1H)-one

To a solution of 4-nitrofenethylamine HCl (3 g, 14.8 mmol) in dimethyl sulfoxide (14.4 mL) was added sodium hydrogen carbonate (6.18 g, 73.56 mmol) and the mixture was stirred for 15 minutes. Then sodium iodie (2.76 g, 18.41 mmol) and (R)-8-(benzyloxy)-5-(2,2-dihydroxyacetyl)quinolin-2(1 H)-one (6 g, 12.28 mmol) were added to the mixture. The reaction was stirred under nitrogen atmosphere at 120 °C for 2 hours.
The crude was partitioned between ether and water and the organic layer was washed several times with water, dried and the solvent was removed under reduced pressure. The crude obtained was purified by column chromatography with silica gel, eluting with chloroform to give the title compound as an orange oil (3.8 g, 47%).

### Intermediate 120. tert-butyl ((2R)-2-[8-(benzyloxy)-2-oxo-1,2-dihydroquinolin-5-yl]-2-{[tert-butyl(dimethyl)silyl]oxy}ethyl)[2-(4-nitrophenyl)ethyl]carbamate

Obtained from Intermediate 119 (3.8 g, 6.62 mmol), di-tert-butyl dicarbonate (1.6 g, 7.33 mmol) and triethylamine (1.01 mL, 7.25 mmol) by the same procedure described in Intermediate 83 (reaction time: 4 hours). The solvent was removed under reduced pressure and the crude was partitioned between ciclohexane:methylen chloride/water. The organic layer was washed several times with water, dried and evaporated to give the title compound as yellow foam (4.35 g, 69%), which was used in the next step without further purification.

### Intermediate 121. tert-butyl [2-(4-aminophenyl)ethyl]((2R)-2-[8-(benzyloxy)-2-oxo-1,2-dihydroquinolin-5-yl]-2-{[tert-butyl(dimethyl)silyl]oxy}ethyl)carbamate

Obtained from Intermediate 120 (4.35 g, 6.46 mmol) and Ni- Raney (1 g, 17.04 mmol) by the same procedure described in Intermediate 84. The solvent was removed under reduced pressure and the title compound was obtained as a solid (3.66 g, 67%) and used in the next step without further purification.

### Intermediate 122. tert-butyl ((2R)-2-[8-(benzyloxy)-2-oxo-1,2-dihydroquinolin-5-yl]-2-{[tert-butyl(dimethyl)silyl]oxy}ethyl)[2-(4-{[(biphenyl-2-ylamino)carbonyl]amino}phenyl)ethyl]carbamate

Obtained from Intermediate 121 (0.9 g, 1.4 mmol) and 2-biphenylisocianate (0.3 g, 1.54 mmol) by the same procedure described in Intermediate 85. The solvent was removed under reduced pressure and the title compound was not isolated and used in the next step without further purification.

### Intermediate 123. N-{4-[2-({(2R)-2-[8-(benzyloxy)-2-oxo-1,2-dihydroquinolin-5-yl]-2-hydroxyethyl}amino)ethyl]phenyl}-N'-biphenyl-2-ylurea

Obtained from Intermediate 122 and acid chloride 4M in dioxane by the same procedure described in Intermediate 86. The crude obtained was purified by column chromatography with silica gel, eluting with chloroform/methanol (8:1) to give the title compound as a solid (0.6 g, 68%).

### EXAMPLE 34. N-biphenyl-2-yl-N'-[4-(2-{[(2R)-2-hydroxy-2-(8-hydroxy-2-oxo-1,2-dihydroquinolin-5-yl)ethyl]amino}ethyl)phenyl]urea

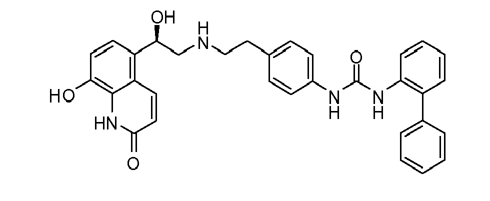

Obtained from Intermediate 123 (0.6 g, 0.96 mmol), palladium on charcoal (0.15 g, 10%) and acid chloride 1.25 M in ethanol (2 mL) by the same procedure described in Example 6. The crude obtained was treated with ethyl acetate giving a solid, which was collected by filtration and washed with acetonitrile to give the title compound as a white solid (0.5 g, 31 %). ¹H-NMR (300 MHz, dimethylsulfoxide-D6): 2.87-3.15 (m, 6H); 5.39 (bs, 1H); 6.15 (bs, 1H); 6.57 (d, *J*=9.89 hz, 1H); 6.99 (d, *J*=8.24 hz, 1H); 7.11-7.22 (m, 5H); 7.3-7.53 (m, 8H); 7.69 (s, 1H); 7.89 (d, *J*=8.51 Hz, 1H); 8.22 (d, *J*=9.89 Hz, 1H); 9.06 (s, 1H); 10.5 (bs, 1H). MS(M+): 535.

### Intermediate 124. tert-butyl ((2R)-2-[8-(benzyloxy)-2-oxo-1,2-dihydroquinolin-5-yl]-2-{[tert-butyl(dimethyl)silyl]oxy}ethyl){2-[4-({[(3-phenylpropyl)amino]carbonyl}amino)phenyl]ethyl}carbamate

Obtained from Intermediate 121 (1 g, 1.55 mmol) and 3-phenylpropyl isocianate (0.27 g, 1.67 mmol) by the same procedure described in Intermediate 85. The solvent was removed under reduced pressure and the crude obtained was used in the next step as a title compound without further manipulation.

### Intermediate 125. N-{4-[2-({(2R)-2-[8-(benzyloxy)-2-oxo-1,2-dihydroquinolin-5-yl]-2-hydroxyethyl}amino)ethyl]phenyl}-N'-(3-phenylpropyl)urea

Obtained from Intermediate 124 and acid chloride 4M in dioxane (90 mL) by the same procedure described in Intermediate 86. The crude obtained was purified by column chromatography with silica gel, eluting with chloroform/methanol (from 10:0 to 8:1) to obtain the title compound as a solid (0.4 g, 44 %).

### EXAMPLE 35. N-[4-(2-{[(2R)-2-hydroxy-2-(8-hydroxy-2-oxo-1,2-dihydroquinolin-5-yl)ethyl]amino}ethyl)phenyl]-N'-(3-phenylpropyl)urea

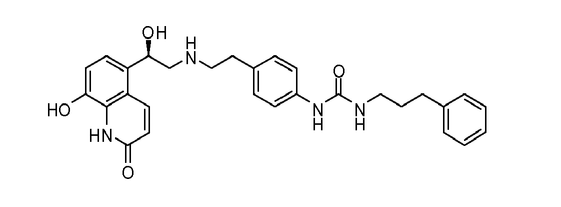

Obtained from Intermediate 125 (0.4 g, 0.68 mmol) and palladium on charcoal (0.08 g, 10%) by the same procedure described in Example 6 (reaction time: 3 hours). The crude obtained was treated with ethyl acetate and the solid was collected by filtration and washed several times with acetonitrile. The title compound was obtained as a solid (0.32 g, 90%). ¹H-NMR (300 MHz, dimethylsulfoxide-D6): 1.71 (q, *J*=7.41 hz, 2H); 2.6 (t, *J*=7.42 Hz, 2H); 2.84-3.18 (m, 8H); 5.42 (bs, 1H); 6.37 (bs, 1H); 6.57 (d, *J*=9.89 Hz, 1H); 7.0 (d, *J*=7.97 Hz, 1H); 7.09 (d, *J*=8.51 Hz, 2H); 7.15-7.31 (m, 6H); 7.35 (d, *J*=8.51 Hz, 2H); 8.23 (d, *J*=9.89 Hz; 1H); 8.65 (bs, 1H); 8.77 (bs, 1H); 9.14 (bs, 1H); 10.51 (bs, 1H). MS(M+): 501.

### Intermediate 126. tert-butyl ((2R)-2-[8-(benzyloxy)-2-oxo-1,2-dihydroquinolin-5-yl]-2-{[tert-butyl(dimethyl)silyl]oxy}ethyl){2-[4-({[(diphenylmethyl)amino]carbonyl}amino)-phenyl]ethyl}carbamate Obtained from Intermediate 121 (0.9 g, 1.4 mmol) and

(isocyanatemethylene)dibenzene (0.3 g, 1.43 mmol) by the same procedure described in Intermediate 85 (reaction time: 40 hours). The solvent was removed under reduced pressure and the solid obtained was treated with hexane. The precipitate was removed by filtratrion and the filtrate was evaporated obtaining the tilte compound, which was used in the next step without further manipulation.

### Intermediate 127. N-{4-[2-({(2R)-2-[8-(benzyloxy)-2-oxo-1,2-dihydroquinolin-5-yl]-2-hydroxyethyl}amino)ethyl]phenyl}-N'-(diphenylmethyl)urea

Obtained from Intermediate 126 and acid chloride 4M in dioxane (50 mL) by the same procedure described in Intermediate 86 (reaction time: 2 hours). The crude obtained was treated with isopropyl ether and the solid obtained was collected by filtratrion and washed with ether to give the title compound as a solid (0.35 g, 36%), which was used in the next step without further manipulation.

### EXAMPLE 36. N-(diphenylmethyl)-N'-[4-(2-{[(2R)-2-hydroxy-2-(8-hydroxy-2-oxo-1,2-dihydroquinolin-5-yl)ethyl]amino}ethyl)phenyl]urea

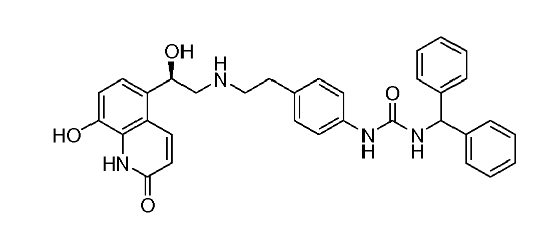

Obtained from Intermediate 127 (0.35 g, 0.52 mmol) and palladium on charcoal (0.07 g, 10%) by the same procedure described in Example 6 (pressure: 35 psi; reaction time: 3 hours). The crude obtained was purified by column chromatography with silica gel, eluting with chloroform/methanol (from 10:0 to 8:1) to give the title compound as a solid (0.12 g, 41%). ¹H-NMR (300 MHz, dimethylsulfoxide-D6): 2.87-3.16 (m, 6H); 5.41 (bs, 1h); 6.00 (d, *J*= 8.24 Hz, 1H); 6.59 (d, *J*=9.61 Hz, 1H); 7.19 (d, *J*=8.55 Hz, 2H); 7.26-7.4 (m, 10H); 7.47 (d, *J*=8.51 Hz, 2H); 8.26 (d, 9.89 Hz, 1H); 8.85 (bs, 1H). MS(M+): 549.

### TEST A

### Human Adrenergic β₁ and β₂ Receptor Binding Assays

The study of binding to human adrenergic ₁ and ₂ receptors was performed using commercial membranes prepared from Sf9 cells where they are overexpressed (Perkin Elmer).

The membrane suspensions (16 µg/well for ₁ and 5µg/well for ₂) in assay buffer, 75mM Tris/HCl with 12.5mM MgCl2 and 2mM EDTA pH=7.4, were incubated with 0.14nM 3H-CGP12177 (Amersham) and different concentrations of the test compounds, in a final volume of 250 µl, in GFC Multiscreen 96 well plates (Millipore) pretreated with + 0.3% PEI. Non specific binding was measured in the presence of 1µM propanolol. Incubation was for 60 minutes at room temperature and with gentle shaking. The binding reactions were terminated by filtration and washing with 2.5 volumes of Tris/HCl 50mM pH=7.4. The affinity of each test compound to the receptor was determined by using at least six different concentrations ran in duplicate. IC₅₀ values were obtained by non-linear regression using SAS.

Selected compounds of the present invention were found to have IC₅₀ values less than 10 nM for β₂ receptor and more than 20 nM for β₁ receptor, with β₁/β₂ ratios from 2,5 to 13.

### TEST B

### Determination of agonist activity and offset of action on isolated guinea-pig tracheal rings (resting tone)

Test compounds and products

The test compounds were dissolved in distilled water. Some of them needed to be dissolved using 10% polyethylene glycol 300 and a few drops of HCl 0.1 N. Isoprenaline hemisulfate (Sigma I 5752) and dissolved in distilled water. Stock solutions were then diluted in Krebs Henseleit solution (NaCl 118mM, KCI 4.7mM, CaCl2 2.52mM, MgSO4 1.66 mM, NaHCO3 24.9mM, KH2PO4 1.18mM, glucose 5.55 mM, sodium pyruvate 2mM) to prepare different concentration ranges per each compound.

### Experimental procedure

The activity of compounds in tracheal ring was assessed according a previously described procedure (Cortijo et al., Eur J Pharmacol. 1991, 198, 171-176). Briefly, adult, male guinea pigs (400-500g) were sacrificed by a blow to the head with immediate exsanguinations (abdominal aorta). Tracheas were excised and placed into Krebs solution in a Petri dish. The adherent connective tissue was dissected away and the lumen gently flushed with Krebs solution. Each trachea was dissected into single rings. First, cotton thread was attached to the cartilage at both sides of the smooth muscle. The rings were opened by cutting through the cartilage on the side opposite to the smooth muscle band. Then, one end of the ring was attached to the strain gauge and the other end was attached to the organ-bath under a resting tension of 1 g and changes in tension of the rings were measured using an isometric transducer. The bath contained Krebs solution gassed with 5% CO2 in oxygen at 37°C. Tissues were then left for one hour to stabilize.

At the beginning of the experience, isoprenaline was administered at a concentration of 0.1µM to test ring relaxation. Rings were then washed twice with Krebs solution and left to recover for 15-30 min. For each compound, a range of increasing and accumulative concentrations (0.01 nM to 0.1 µM) was administered with a maximum waiting time of 30 min between each administration. After the maximum concentration (achievement of complete relaxation), ring preparations were washed every 15 min during 1 hour. At the end of the experiment, 0.1 µM of isoprenaline was administered to each preparation to produce maximum relaxation back.

### Determination of agonist activity and offset of action

Agonist activity was determined by assaying accumulative increasing concentrations of test compounds prepared in the Krebs solution. The magnitude of each response was measured and expressed as a percentage versus the maximum relaxation induced by isoprenaline. Potency values for the test compounds were expressed in absolute terms (concentration required to induce a 50% relaxation, EC50).
The time to 50% offset of action is defined as the time from the end of test compounds administration to attainment of 50% recovery. Recovery time was expressed as the percentage of recovery (loss of relaxation) reached 1 h after test compounds administration.

Selected compounds of this invention showed EC₅₀ values less than 1 nM with less than 10 % recovery at 60 min.

### TEST C

### Acetylcholine-induced bronchoconstriction in guinea pig

Test compounds and products

The test compounds were dissolved in distilled water. Some of them need to be dissolved using a maximum of 10% polyethylene glycol 300. Acetylcholine HCl was supplied by Sigma (code A 6625) and dissolved in saline solution.

### Experimental procedure

Male guinea-pigs (450-600g) were supplied by Harlan (Netherlands), and maintained at a constant temperature of 22±2 °C, humidity 40-70% with 10 cycles of room air per hour. They were illuminated with artificial light in 12 hour cycles (from 7h am to 7h pm). A minimum of 5 days acclimatization period was left before animals were dosed with test compounds. The animals were fasted 18 hours before the experiment with water ad libitum.

Guinea pigs were exposed to an aerosol of a test compound or vehicle. These aerosols were generated from aqueous solutions using a Devilbiss nebuliser (Model Ultraneb 2000, Somerset, PA, SA). A mixture of gases (CO2=5%, O2=21%, N2=74%) was flown through the nebuliser at 3 L/minute. This nebuliser was connected to a methacrylate box (17x17x25 cm) where the animals were placed one per session. Every guinea pig remained in the box for a total of 10 minutes. Aerosols were generated at 0 and 5 minutes during 60 seconds each one (approximately 5 mL of solution was nebulised).

Aerosol concentrations between 0.1 and 300 µg/ml of the compounds were administered. The bronchoprotective effects of test compounds were evaluated one hour or twenty four hours post-dose with a Mumed PR 800 system.

### Determination of bronchoprotective effect and calculations

The guinea pigs were anesthetized with an intramuscular injection of ketamine (43.75 mg/kg), xylazine (83.5 mg/kg), and acepromazine (1.05 mg/kg) at a volume of 1 ml/kg. After the surgical site was shaved, a 2-3 cm midline incision of the neck was made. The jugular vein was isolated and cannulated with a polyethylene catheter (Portex Ld.) to allow an intravenous bolus of acetylcoline (10 and 30 µg/kg iv) at 4-min intervals. The carotid artery was cannulated and the blood pressure was measured by a Bentley Tracer transducer. The trachea was dissected and cannulated with a teflon tube and connected at a pneumotachograph Fleisch for measuring the airflow. Animal was ventilated using an Ugo Basile pump, with a volume of 10 ml/kg at a rate of 60 breaths/min. The transpulmonary pressure was measured with an esophageal cannula (Venocath-14, Venisystems) connected to Celesco transducer. Once the cannulations were completed a Mumed pulmonary measurement computer program enabled the collection of pulmonary values. The baseline values were within the range of 0.3-0.9 mL/cm H2O for compliance and within the range of 0.1-0.199 cm H2O/mL per second for lung resistance (RL).

The bronchocoprotective effect of inhaled compounds was determined with the concentration of the test compound causing a 50 % of inhibition of bronchoconstriction (EC50) induced by acetylcholine at 30 µg/kg iv

Determination of duration of action

Selected compounds of this invention show long duration of action with a ratio ED₅₀ at 24 hr/ED₅₀ at 4 hr less than 3.

### Pharmaceutical Compositions

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule. Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred.

Each capsule or cartridge may generally contain between 2µg and 150 µg of each therapeutically active ingredient. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices.
The powder formulation has to exhibit good and stable flow properties, because filling of the dose measuring cups or cavities is mostly under the influence of the force of gravity. For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with.

Such atomisers are described, for example, in PCT Patent Application No. W0 91/14468 and International Patent Application No. WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate. Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit contains suitably from 1 µg to 100 µg, and preferably from 5 µg to 50 µg of a β2-agonist according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day.

Examples of suitable PDE4 inhibitors that can be combined with β2-agonists are denbufylline, rolipram, cipamfylline, arofylline, filaminast, piclamilast, mesopram, drotaverine hydrochloride, lirimilast, roflumilast, cilomilast, oglemilast, apremilast, 6-[2-(3,4-Diethoxyphenyl)thiazol-4-yl]pyridine-2-carboxylic acid (tetomilast), (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine, N-(3,5-Dichloro-4-pyridinyl)-8-methoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2-methoxyethyl)pyridin-2(1H)-one, 2-carbomethoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent applications number WO03/097613, WO2004/058729, WO 2005/049581, WO 2005/123693 and WO 2005/123692

Examples of suitable corticosteroids and glucocorticoids that can be combined with β2-agonists are prednisolone, methylprednisolone, dexamethasone, Dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, Butixocort propionate, RPR-106541, halometasone, methylprednisolone suleptanate, mometasone furoate, rimexolone, prednisolone farnesylate, ciclesonide, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable M3 antagonists (anticholinergics) that can be combined with β2-agonists are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts, 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-1 04135), 2(R)-Cyclopentyl-2-hydroxy-N-[1-[4(S)-methylhexyl]piperidin-4-yl]-2-phenylacetamide (J-106366), 2(R)-Cydopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Particularly preferred pharmaceutical composition according to the invention comprise a compound of formula (I) and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts, 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO03/097613, WO2004/058729, WO 2005/049581, WO 2005/123693 and WO 2005/123692

Thus, in one aspect of the invention, the composition comprises a compound of formula (I) and a corticosteroid. Particularly preferred corticosteroids are those selected from the group consisting of mometasone furoate, ciclesonide, budesonide, fluticasone furoate and fluticasone propionate.

In another aspect of the invention, the composition comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. The composition may further comprise a corticosteroid selected from the group consisting of mometasone furoate, ciclesonide, budesonide, fluticasone furoate and fluticasone propionate.

In a still other aspect of the invention, the composition comprises a compound of formula (I) and a PDE4 inhibidor. Particularly preferred PDE4 inhibidors are those selected from the group consisting of rolipram, roflumilast, cilomilast and the compounds claimed in the PCT patent applications number WO03/097613, WO2004/058729, WO 2005/049581, WO 2005/123693 and WO 2005/123692. The composition may further comprise a corticosteroid selected from the group consisting of mometasone furoate, ciclesonide, budesonide, fluticasone furoate and fluticasone propionate. In addition to the salt of the invention and to the PDE4 inhibitor, the composition may further comprise an anticholinergic agent selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

In a preferred embodiment of the present invention, the composition comprises a compound of formula (I) and a therapeutically effective amount of a 3-[2-Hydroxy-2,2-bis(2-thienyl)acetoxy]-1-(3-phenoxypropyl)-1-azoniabicyclo[2.2.2]octane salts. Optionally, the composition further comprises a corticosteroid and/or a PDE4 inhibidor.

In another preferred embodiment of the present invention, the composition comprises a compound of formula (I) and a therapeutically effective amount of a mometasone furoate. Optionally, the composition further comprises an anticholinergic salt and/or a PDE4 inhibidor.

In another embodiment of the invention, the composition comprises a compound of formula (I), a corticosteroid, an anticholinergic agent and a PDE4 inhibidor.

The combinations of the invention may be used in the treatment of respiratory diseases, wherein the use of bronchodilating agents is expected to have a beneficial effect, for example asthma, acute or chronic bronchitis, emphysema, or Chronic Obstructive Pulmonary Disease (COPD).

The active compounds in the combination, i.e. the β2-agonist of the invention and the PDE4 inhibitors, corticosteroids or glucocorticoids and/or anticholinergics may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

Additional suitable carriers for formulations of the active compounds of the present invention can be found in Remington: The Science and Practice of Pharmacy, 20th Edition, Lippincott Williams & Wilkins, Philadelphia, Pa., 2000. The following nonlimiting examples illustrate representative pharmaceutical compositions of the invention.

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 ml |

## Claims

1. A compound of formula (I): 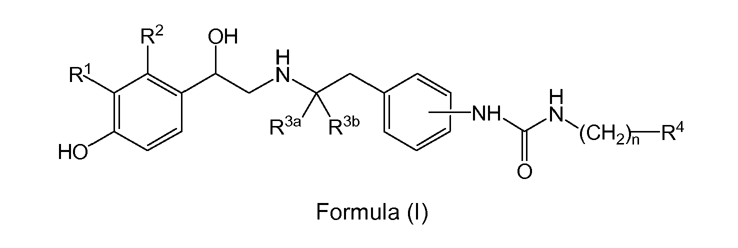 wherein:
R¹ is a group selected from -CH₂OH,-NHCOH and
R² is a hydrogen atom; or
R¹ together with R² form the group -NHC(O)CH=CH-, wherein the nitrogen atom is bound to the carbon atom in the phenyl ring holding R¹ and the carbon atom is bound to the carbon atom in the phenyl ring holding R²,
R^{3a} and R^{3b} are independently selected from the group consisting of hydrogen atoms and C₁₋₄ alkyl groups
n is an integer selected from 0 to 6,
R⁴ is selected from the group consisting of an optionally substituted monocyclic or polycyclic C₃₋₁₀ cycloalkyl group, an optionally substituted monocyclic C₅₋₁₀ aryl group and a methyl group which is substituted with one or more substituents selected from C₅₋₁₀ aryl and C₅₋₁₀ aryloxy groups,
wherein the monocyclic or polycyclic C₃₋₁₀ cycloalkyl and the monocyclic C₅₋₁₀ aryl groups independently are optionally substituted with one or more substituents selected from halogen atoms, C₁₋₄ alkyl, C₁₋₄ alkoxy, C₅₋₁₀ aryl and C₅₋₁₀ aryloxy groups, or a pharmaceutically-acceptable salt or solvate or stereoisomer thereof.

2. A compound according to claim 1 wherein R¹ together with R² form the group - NHC(O)CH=CH-, wherein the nitrogen atom is bound to the carbon atom in the phenyl ring holding R¹ and the carbon atom is bound to the carbon atom in the phenyl ring holding R².

3. A compound according to any preceding claim wherein R^{3a} and R^{3b} are independently selected from the group consisting of hydrogen atoms and methyl groups.

4. A compound according to claim 3 wherein R^{3a} represents a hydrogen atom and R^{3b} is selected from the group consisting of hydrogen atoms and methyl groups

5. A compound according to any preceding claim wherein n has a value from 0 to 3.

6. A compound according to claim 5 wherein n has a value of 0 or 1.

7. A compound according to any preceding claim wherein R⁴ is selected from the group consisting of monocyclic or polycyclic C₄₋₁₀ cycloalkyl, a phenyl group and a methyl group which is substituted with one or two substituents selected from a phenyl group and a phenyloxy group and wherein the cycloalkyl and the phenyl groups independently are optionally substituted with one or more substituents selected from fluorine and chlorine atoms, methoxy, phenyl and phenoxy groups.

8. A compound according to claim 7 wherein R⁴ is selected from the group consisting of cyclohexyl, 1-adamantyl, phenyl and -CH(Ph)₂ groups, wherein the phenyl group is optionally substituted with one or more substituents selected from methoxy, phenyl and phenoxy groups.

9. A compound according to claim 8, wherein R⁴ is selected from the group consisting of a phenyl and -CH(Ph)₂ groups, wherein the phenyl group is optionally substituted with one or two substituents selected from methoxy, and phenyl groups.

10. A compound according to any preceding claim having a formula (IA): 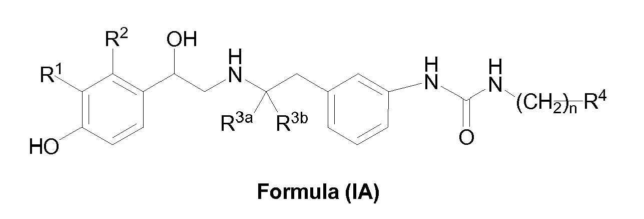 Wherein R¹, R², R^{3a}, R^{3b}, R⁴ and n are as defined in anyone of the preceding claims

11. A compound according to any preceding claim having a formula (IA): 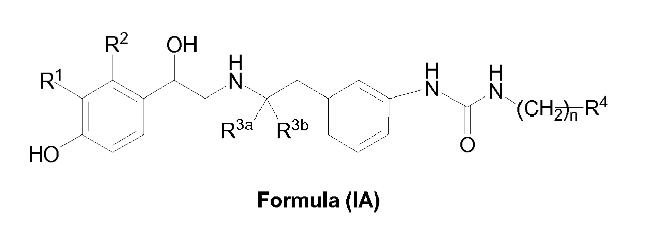 wherein:
R¹ together with R² form the group -NHC(O)CH=CH-, wherein the nitrogen atom is bound to the carbon atom in the phenyl ring holding R¹ and the carbon atom is bound to the carbon atom in the phenyl ring holding R²,
R^{3a} represents a hydrogen atom and R^{3b} is selected from the group consisting of hydrogen atom and a methyl group, and
n has a value of 0 or 1, and
R⁴ is selected from the group consisting of a phenyl group and a -CH(Ph)₂ group,
wherein the phenyl group is optionally substituted with one or two substituents selected from methoxy, and phenyl groups.

12. A compound according to claim 1 which is one of:
N-benzyl-N'-{3-[(2*R,S*)-2-({(2*R,S*)-2-hydroxy-2-[4-hydroxy-3-(hydroxymethyl)-phenyl]ethyl}amino)propyl]phenyl}urea
N-benzyl-N'-{4-[(2*R,S*)-2-({(2*R,S*)-2-hydroxy-2-[4-hydroxy-3-(hydroxymethyl)-phenyl]ethyl}amino)propyl]phenyl}urea
N-benzyl-N'-[3-((2*R,S*)-2-{[(2*R,S*)-2-hydroxy-2-(8-hydroxy-2-oxo-1,2-dihydroquinolin-5-yl)ethyl]amino}propyl)phenyl]urea
N-{3-[(2*R,S*)-2-({(2*R,S*)-2-hydroxy-2-[4-hydroxy-3-(hydroxymethyl)phenyl]-ethyl}amino)propyl]phenyl}-N'-(2-methoxybenzyl)urea
N-(2, 6-dimethoxybenzyl)-N'-{3-[(2*R,S*)-2-({(2*R*)-2-hydroxy-2-[4-hydroxy-3-(hydroxymethyl)phenyl]ethyl}amino)propyl]phenyl}urea
N-[3-((2*R,S*)-2-{[(2*R,S*)-2-hydroxy-2-(8-hydroxy-2-oxo-1,2-dihydroquinolin-5-yl)ethyl]amino}propyl)phenyl]-N'-(2-methoxybenzyl)urea
N-benzyl-N'-{3-[2-({(2R,S)-2-hydroxy-2-[4-hydroxy-3-(hydroxymethyl)phenyl]-ethyl}amino)ethyl]phenyl}urea
N-benzyl-N'-[3-(2-{[(2*R,S*)-2-hydroxy-2-(8-hydroxy-2-oxo-1,2-dihydroquinolin-5-yl)ethyl]amino}ethyl)phenyl]urea
N-[3-(2-{[(2*R,S*)-2-hydroxy-2-(8-hydroxy-2-oxo-1,2-dihydroquinolin-5-yl)ethyl]-amino}ethyl)phenyl]-N'-(2-methoxybenzyl)urea
N-(4-fluorobenzyl)-N'-[3-(2-{[(2*R,S*)-2-hydroxy-2-(8-hydroxy-2-oxo-1,2-dihydroquinolin-5-yl)ethyl]amino}ethyl)phenyl]urea
N-[3-(2-{[(2*R,S*)-2-hydroxy-2-(8-hydroxy-2-oxo-1,2-dihydroquinolin-5-yl)ethyl]-amino}ethyl)phenyl]-N'-(2-phenylethyl)urea
N-[3-(2-{[(2*R,S*)-2-hydroxy-2-(8-hydroxy-2-oxo-1,2-dihydroquinolin-5-yl)ethyl]-amino}ethyl)phenyl]-N'-(3-phenylpropyl)urea
N-{3-[2-({(2*R,S*)-2-hydroxy-2-[4-hydroxy-3-(hydroxymethyl)phenyl]ethyl}-amino)ethyl]phenyl}-N'-(3-phenylpropyl)urea
N-(diphenylmethyl)-N'-[3-(2-{[(2*R,S*)-2-hydroxy-2-(8-hydroxy-2-oxo-1,2-dihydroquinolin-5-yl)ethyl]amino}ethyl)phenyl]urea
N-[3-(2-{[(2*R,S*)-2-hydroxy-2-(8-hydroxy-2-oxo-1,2-dihydroquinolin-5-yl)ethyl]-amino}ethyl)phenyl]-N'-phenylurea
N-1-adamantyl-N'-[3-(2-{[(2*R,S*)-2-hydroxy-2-(8-hydroxy-2-oxo-1,2-dihydroquinolin-5-yl)ethyl]amino}ethyl)phenyl]urea
N-(2,6-dichlorobenzyl)-N'-[3-(2-{[(2*R,S*)-2-hydroxy-2-(8-hydroxy-2-oxo-1,2-dihydroquinolin-5-yl)ethyl]amino}ethyl)phenyl]urea
N-biphenyl-2-yl-N'-[3-(2-{[(2*R,S*)-2-hydroxy-2-(8-hydroxy-2-oxo-1,2-dihydroquinolin-5-yl)ethyl]amino}ethyl)phenyl]urea
N-cyclohexyl-N'-[3-(2-{[(2*R,S*)-2-hydroxy-2-(8-hydroxy-2-oxo-1,2-dihydroquinolin-5-yl)ethyl]amino}ethyl)phenyl]urea
N-[3-(2-{[(2*R,S*)-2-hydroxy-2-(8-hydroxy-2-oxo-1,2-dihydroquinolin-5-yl)ethyl]-amino}ethyl)phenyl]-N'-(4-phenylbutyl)urea
N-[3-(2-{[(2*R,S*)-2-hydroxy-2-(8-hydroxy-2-oxo-1,2-dihydroquinolin-5-yl)ethyl]-amino}ethyl)phenyl]-N'-(4-methoxyphenyl)urea
N-biphenyl-4-yl-N'-[3-(2-{[(2*R,S*)-2-hydroxy-2-(8-hydroxy-2-oxo-1,2-dihydroquinolin-5-yl)ethyl]amino}ethyl)phenyl]urea
N-(1-adamantylmethyl)-N'-[3-(2-{[(2*R,S*)-2-hydroxy-2-(8-hydroxy-2-oxo-1,2-dihydroquinolin-5-yl)ethyl]amino}ethyl)phenyl]urea
N-[3-(2-{[(2*R,S*)-2-hydroxy-2-(8-hydroxy-2-oxo-1,2-dihydroquinolin-5-yl)ethyl]-amino}ethyl)phenyl]-N'-(3-phenoxyphenyl)urea
N-[3-(2-{[(2*R*)-2-hydroxy-2-(8-hydroxy-2-oxo-1,2-dihydroquinolin-5-yl)ethyl]-amino}ethyl)phenyl]-N'-(3-phenylpropyl)urea
N-(diphenylmethyl)-N'-[3-(2-{[(2*R*)-2-hydroxy-2-(8-hydroxy-2-oxo-1,2-dihydroquinolin-5-yl)ethyl]amino}ethyl)phenyl]urea
*N*-biphenyl-2-yl-N'-[3-(2-{[(2*R*)-2-hydroxy-2-(8-hydroxy-2-oxo-1,2-dihydroquinolin-5-yl)ethyl]amino}ethyl)phenyl]urea
*N*-biphenyl-2-yl-N'-{3-[2-({(2*R*)-2-[3-(formylamino)-4-hydroxyphenyl]-2-hydroxyethyl}amino)ethyl]phenyl}urea
*N*-biphenyl-2-yl-*N'*-[3-(2-{[(2*R*)-2-hydroxy-2-(8-hydroxy-2-oxo-1,2-dihydroquinolin-5-yl)ethyl]amino}propyl)phenyl]urea, (isomer A)
*N*-biphenyl-2-yl-*N'*-{3-[2-({(2*R*)-2-hydroxy-2-[4-hydroxy-3-(hydroxymethyl)phenyl]-ethyl}amino)propyl]phenyl}urea, (isomer B)
*N*-biphenyl-2-yl-*N'*-{3-[2-({(2*R*)-2-hydroxy-2-[4-hydroxy-3-(hydroxymethyl)phenyl]-ethyl}amino)propyl]phenyl}urea, (isomer A)
*N*-biphenyl-2-yl-*N'*-[3-(2-{[(2*R*)-2-hydroxy-2-(8-hydroxy-2-oxo-1,2-dihydroquinolin-5-yl)ethyl]amino}propyl)phenyl]urea, (isomer B)
*N*-(4-tert-butylbenzyl)-*N'*-[3-(2-{[(2*R,S*)-2-hydroxy-2-(8-hydroxy-2-oxo-1,2-dihydro-quinolin-5-yl)ethyl]amino}ethyl)phenyl]urea
*N*-biphenyl-2-yl-*N'*-[4-(2-{[(2*R*)-2-hydroxy-2-(8-hydroxy-2-oxo-1,2-dihydroquinolin-5-yl)ethyl]amino}ethyl)phenyl]urea
*N*-[4-(2-{[(2*R*)-2-hydroxy-2-(8-hydroxy-2-oxo-1,2-dihydroquinolin-5-yl)ethyl]amino}-ethyl)phenyl]-*N'*-(3-phenylpropyl)urea *N*-(diphenylmethyl)-*N'*-[4-(2-{[(2*R*)-2-hydroxy-2-(8-hydroxy-2-oxo-1,2-dihydro-quinolin-5-yl)ethyl]amino}ethyl)phenyl]urea and pharmaceutically-acceptable salts and solvates thereof.

13. A pharmaceutical composition comprising a therapeutically effective amount of a compound according to anyone of claims 1 to 12 and a pharmaceutically acceptable carrier.

14. The pharmaceutical composition according to claim 13, wherein the composition further comprises a therapeutically effective amount of one or more other therapeutic agents.

15. The pharmaceutical composition according to claim 14 wherein the other therapeutic agent is a corticosteroid, an antichlolinergic agent, and/or a PDE4 inhibitor.

16. The pharmaceutical composition according to anyone of claims 13 to 15, wherein the composition is formulated for administration by inhalation.

17. A combination comprising a compound according to anyone of claims 1 to 12 and one or more other therapeutic agents.

18. The combination according to claim 17 wherein the other therapeutic agent is a corticosteroid, an antichlolinergic agent, and/or a PDE4 inhibitor.

19. A method of treating a disease or condition in a mammal associated with β2 adrenergic receptor activity, the method comprising administering to the mammal, a therapeutically effective amount of a compound according to any one of claims 1-12 or a pharmaceutical composition of any one of claims 13 to 16.

20. The method according to claim 19 wherein the disease or condition is a pulmonary disease.

21. The method according to claim 20 wherein the pulmonary disease is asthma or chronic obstructive pulmonary disease.

22. The method according to claim 19 wherein the disease or condition is selected from the group consisting of pre-term labor, glaucoma, neurological disorders, cardiac disorders, and inflammation.

23. The method according to anyone of claims 19 to 22 which method further comprises administering a therapeutically effective amount of one or more other therapeutic agents.

24. The method according to claim 23 wherein the other therapeutic agent is a corticosteroid, an anticholinergic agent, and/or a PDE4 inhibitor.

25. Use of a compound according to any one of claims 1 to 12 or a pharmaceutical composition according to any one of claims 13 to 16 in the manufacture of a medicament for the treatment of a disease or condition in a mammal.

26. Use according to claim 25, wherein the disease or condition is a pulmonary disease.

27. Use according to claim 26, wherein the pulmonary disease is asthma or chronic obstructive pulmonary disease.

28. Use according to claim 25, wherein the desease is selected from the group consisting of pre-term labor, glaucoma, neurological disorders, cardiac disorders, and inflammation

29. A compound according to any one of claims 1 to 12 or a pharmaceutical composition according to any one of claims 13 to 16 for use in the treatment of a disease or condition.

30. A compound according to claim 29, wherein the disease or condition is a pulmonary disease

31. A Compound according to claim 30, wherein the pulmonary disease or condition is asthma or chronic obstructive pulmonary disease.

32. A Compound according to claim 29, wherein the disease or condition is is selected from the group consisting of pre-term labor, glaucoma, neurological disorders, cardiac disorders, and inflammation.
